# EUROPEAN PATENT APPLICATION

(11) **EP 3 482 796 A2**
(43) Date of publication of application: **15.05.2019**
(21) Application number: 18205282.9
(22) Date of filing: 08.11.2018
(51) Int. Cl.: A61N 2/00, A61N 2/02

(54) **PORTABLE REPETITIVE TRANSCRANIAL MAGNETIC STIMULATION APPARATUS**

(30) Priority: 09.11.2017 TW 10616677 U; 14.02.2018 TW 10705606
(71) Applicant: Tong, Han, New Taipei (TW)
(72) Inventor: Tong, Han, New Taipei (TW)
(74) Representative: Michalski Hüttermann & Partner Patentanwälte mbB

(57) **Abstract**

A portable repetitive transcranial magnetic stimulation (rTMS) apparatus is provided. The rTMS apparatus includes an upper fastening component, a lower fastening component, a driver circuit and an inductor which is electrically connected to the driver circuit and used as a stimulator. The inductor is formed of a core and a coil. The core has a groove. The coil includes an upper part and a lower part. The upper part of the coil is configured to be distal to the core and pass through an upper side, a left side or a right side of the core. The lower part of the coil is configured to pass through the groove.

## Description

### [FIELD OF THE INVENTION]

The present invention relates to a portable repetitive transcranial magnetic stimulation (rTMS) apparatus. Specifically, the portable rTMS apparatus of the present invention controls operations of driver circuit loop respectively via pulse generators, and a specific designed stimulator is used in combination with the portable rTMS apparatus to achieve the purpose of being portable.

### [DESCRIPTION OF THE RELATED ART]

A common repetitive transcranial magnetic stimulation (rTMS) apparatus currently available has a relatively large volume and must be connected to the wall outlet when using, so the use thereof is limited by location and in most cases, a patient can only receive the rTMS treatment at the location where the rTMS apparatus is located. Moreover, the stimulator of the rTMS apparatus will generate noise during the operation, and the temperature of the stimulator also increases gradually as the operating time increases so that the stimulator cannot be used if the temperature thereof is too high.

Accordingly, an urgent need exists in the art to provide a portable rTMS apparatus which can be used, while not limited by location.

### [CONTENTS OF THE INVENTION]

An objective of the present invention is to provide a portable repetitive transcranial magnetic stimulation (rTMS) apparatus, cooperate with the newly designed stimulator (an inductor) and circuit to remarkably reduce the overall volume of the apparatus, and further reduce the power consumption and achieving the feature of being portable. Accordingly, as compared to the conventional rTMS apparatus, the portable rTMS apparatus of the present invention is not limited by location when using, and can be powered by a built-in battery or a mobile power supply.

To achieve the aforesaid objective, the present invention discloses a portable rTMS apparatus, which comprises a driver circuit and an inductor. The inductor is electrically connected to the driver circuit and is used as a stimulator. The inductor includes a core and at least one coil. The core has a groove. The at least one coil has an upper part and a lower part. The upper part of the at least one coil is configured to be distant to the core and pass through the upper side, the left side or the right side of the core. The lower part of the at least one coil is configured to pass through the groove of the core.

After reading the preferred embodiments and the appended drawings, people skilled in this field may understand the technical features, implementation or other purpose of the claimed invention.

### [BRIEF DESCRIPTION OF THE DRAWINGS]

**FIG. 1** is a schematic view depicting the appearance of a portable repetitive transcranial magnetic stimulation (rTMS) apparatus **1** within the present invention;
**FIG. 2A** is a schematic view depicting a cross-sectional structure of a core **COE**;
**FIG. 2B** is a schematic view depicting a structure of the core **COE**;
**FIG. 2C** to **FIG. 2F** are schematic views depicting the cross-sectional view of the core **COE** and the arrangement of a lower part of the coil;
**FIG. 2G** to **FIG. 2H** are schematic views depicting the bending and extending of the core **COE**;
**FIG. 2I** is a schematic view depicting the core **COE**;
**FIG. 3A** is a view depicting the magnetic flux distribution of the core **COE**;
**FIG. 3B** to **FIG. 3D** depict the arrangement of an upper part of a coil **COL** and a magnetic field distribution simulated diagram of the core **COE**;
**FIG. 3E** to **FIG. 3F** depict the shape of the upper part of the coil **COL** and the magnetic field distribution simulated diagram of the core **COE**;
**FIG. 4A** to **FIG. 4C** are schematic views depicting the arrangement of the upper part of the coil **COL**;
**FIG. 5A** to **FIG. 5C** are schematic architectural views depicting an upper fastening component **UFC** and a lower fastening component **LFC**;
**FIG. 6A** is a schematic structural view depicting a ceramic substrate;
**FIG. 6B** is a schematic view depicting the arrangement of ceramic substrates;
**FIG. 7A** to **FIG. 7C** are schematic views depicting structural relationships between ceramic substrates and extended ceramic substrates;
**FIG. 8A** is a schematic view depicting the arrangement of an upper blocking component;
**FIG. 8B** is a schematic view depicting the arrangement of a lower blocking component and a magnetostrictive material;
**FIG. 9A** to **FIG. 9E** are schematic views depicting the deformation of the core **COE**;
**FIG. 10** is a schematic view depicting an inductor disposed within a housing;
**FIG. 11A** and **FIG. 11B** are schematic views depicting the circuit of the portable rTMS apparatus **1** within the present invention;
**FIG. 12A** depicts the changing current of inductor **L** when charging and discharging;
**FIG. 12B** depicts the current direction of the inductor **L** within a time **T1**;
**FIG. 12C** depicts the current direction of the inductor **L** within a time **T2**;
**FIG. 12D** depicts the current direction of the inductor **L** within a time **T3**;
**FIG. 12E** depicts the current direction of the inductor **L** within a time **T4**;
**FIG. 13** to **FIG. 14** depict schematic views of circuits of the portable rTMS apparatus **1** for different embodiments within the present invention;
**FIG. 15A** to **FIG. 15C** are schematic views depicting the passive snubber **SB**;
**FIG. 16A** to **FIG. 16C**, **FIG. 17A** to **FIG. 17B** and **FIG. 18** to **FIG. 35** are schematic views depicting the circuit of the portable rTMS apparatuses **1** according to different embodiments within the present invention;
**FIG. 36A** is a schematic view depicting circuits connected in parallel of the portable rTMS apparatus **1** within the present invention;
**FIG. 36B** is a schematic view depicting circuits connected in series of the portable rTMS apparatus **1** within the present invention;
**FIG. 36C** is a schematic view depicting circuits connected in series and in parallel of the portable rTMS apparatus **1** within the present invention; and

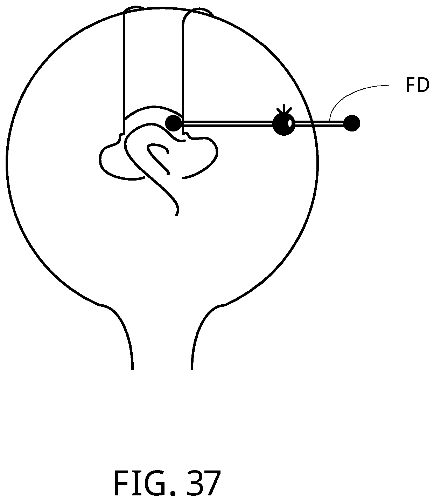

**FIG. 37** is a schematic view depicting a fastening device within the present invention.

### [DESCRIPTION OF EMBODIMENTS]

In the following description, the present invention will be explained with reference to embodiments thereof. It shall be appreciated that, these embodiments within the present invention are not intended to limit the present invention to any particular environment, applications or special mode described in these embodiments. Therefore, description of these embodiments is only for purpose of illustration rather than to limit the present invention, and the scope claimed in this application shall be governed by the claims. Besides, in the following embodiments and the attached drawings, elements unrelated to the present invention are omitted and not depicted; and dimensional relationships among individual elements in the attached drawings are illustrated only for ease of understanding, but not to limit the actual scale. Unless otherwise defined, material properties are all measured at a room temperature.

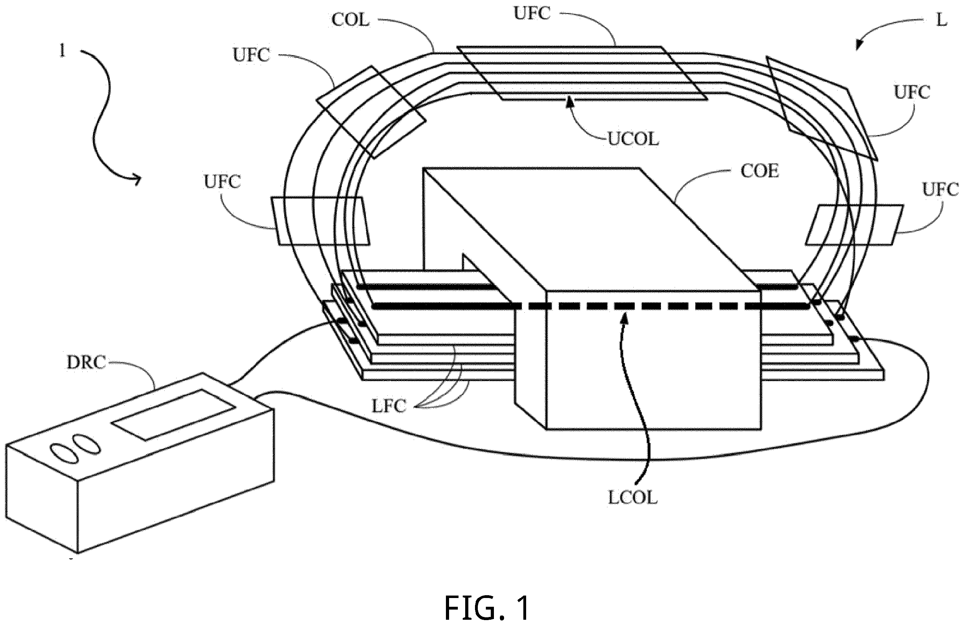

A first embodiment of the present invention is as shown in **FIG. 1** to **FIG. 10**. **FIG. 1** is a schematic view depicting the appearance of a portable repetitive transcranial magnetic stimulation (rTMS) apparatus **1** within the present invention. The portable rTMS apparatus comprises an upper fastening component **UFC**, a lower fastening component **LFC**, a driver circuit **DRC** and an inductor **L**. The driver circuit **DRC** may be encapsulated within a housing, and the inductor **L** may be disposed within another housing (not shown). A controller, a screen, a button and a switch may be additionally provided on the housing of the driver circuit **DRC**. The portable rTMS apparatus may be turned on, turned off or perform other operations via the controller. The controller may respond to actions of the user, such as operating the button, operating the switch, touching the screen or other related operation.

The inductor **L** is electrically connected to the driver circuit **DRC** and is used as a stimulator. The inductor **L** includes a core **COE** and at least one coil **COL**. The core **COE** has a groove **CG**, a coil **COL** has an upper part **UCOL**, a lower part **LCOL** and two connecting portions **INT**. The upper part **UCOL** of the coil **COL** is configured to be distant to the core **COE** and pass through an upper side, a left side or a right side of the core **COE**, and is fastened by the upper fastening component **UFC**. The lower part **LCOL** of the coil **COL** is configured to pass through the groove **CG** and is fastened by the lower fastening component **LFC**.

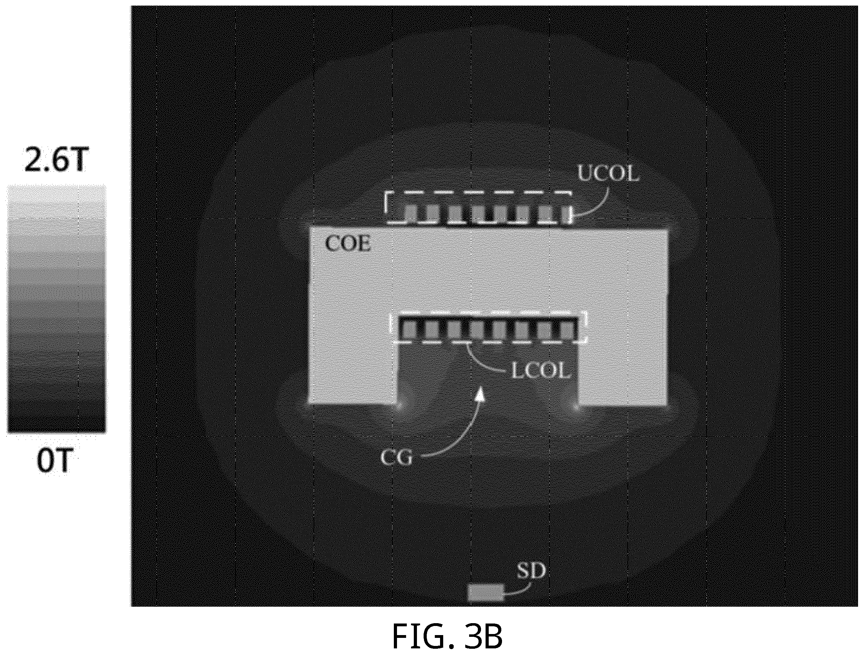

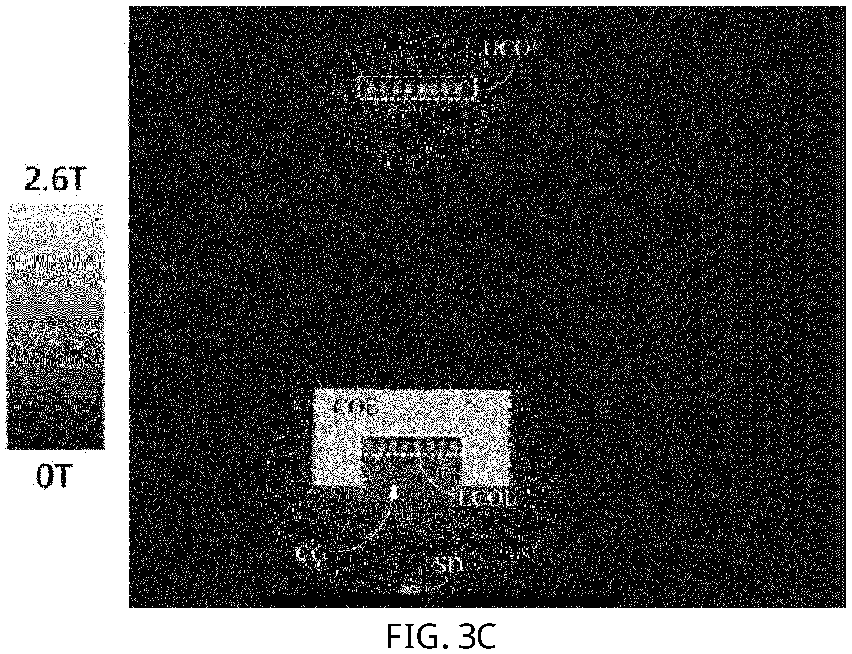

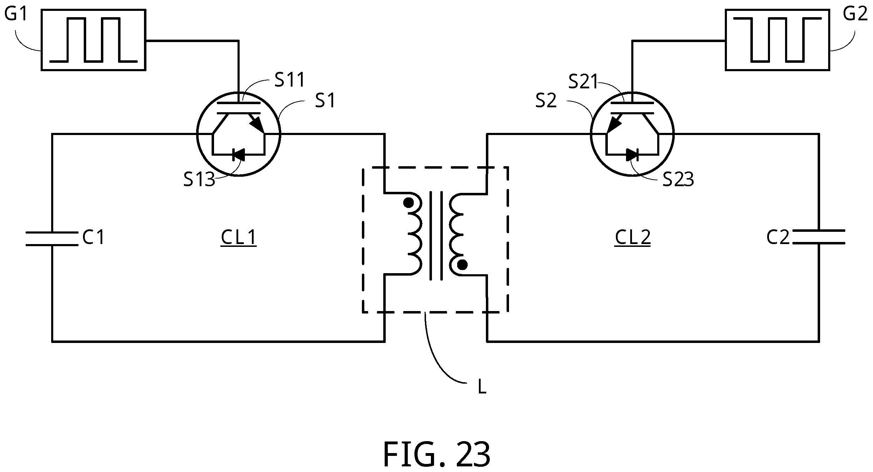

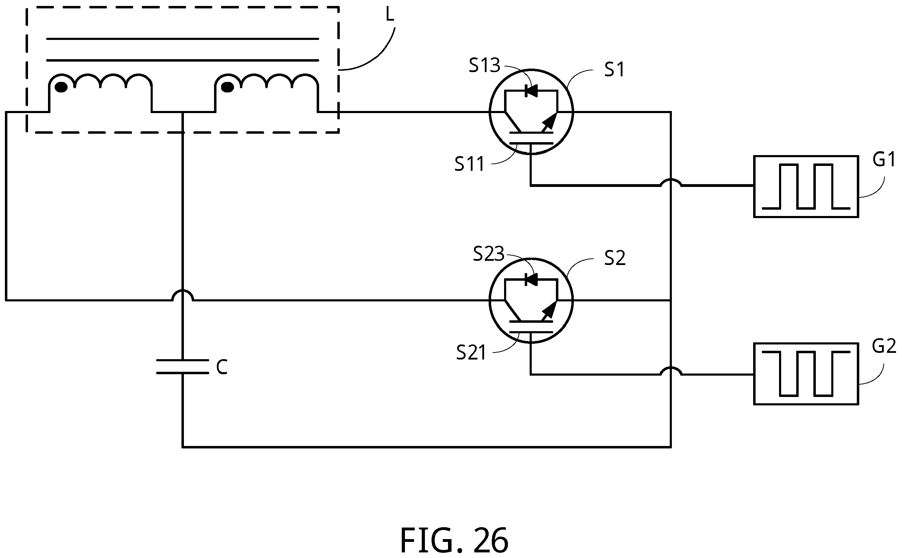

Concretely speaking, as shown in **FIG. 3B** to **FIG. 3F**, the coil **COL** that is located within some two section plane of the core **COE** are the upper part **UCOL** and the lower part **LCOL**, respectively. The lower part **LCOL** is the stimulating portion of the stimulator, and the upper part **UCOL** of the coil **COL** is responsible for flowing back the current of the lower part **LCOL**. Therefore, the current direction of the upper part **UCOL** is opposite to the current direction of the lower part **LCOL**. For example, as seen from the section plane of the core of **FIG. 3C**, if the direction of the lower part **LCOL** is in-to-plane, then the direction of the upper part **UCOL** is out-of-plane. In addition, conductors outside the some two section plane of the core **COE** (i.e., conductors of the connecting portion **INT**, which do not belong to the conductors of the upper part **UCOL** and the lower part **LCOL**) belong to the transitional conductors. A single coil should comprise at least two connection terminals. If two coils are winded on the core **COE**, and four connection terminals of the two coils are not connected with each other, then the two coils can be regarded as two inductors with mutual coupling, as shown in **FIG. 23**, **FIG. 24** and **FIG. 25**. If at least two coils are winded on the core **COE**, and two terminals of different coils are connected, then the coils can be regarded as a center-tapped inductor with three connection terminals, as shown in **FIG. 26**, **FIG. 27**, **FIG. 28**, **FIG. 29**, **FIG. 30** and **FIG. 31**.

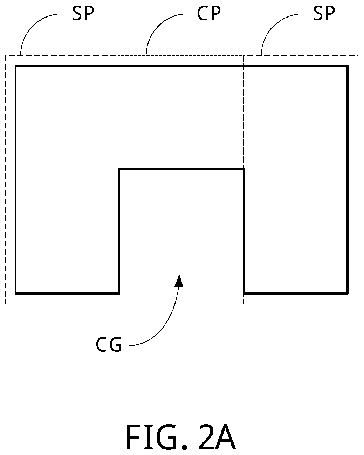

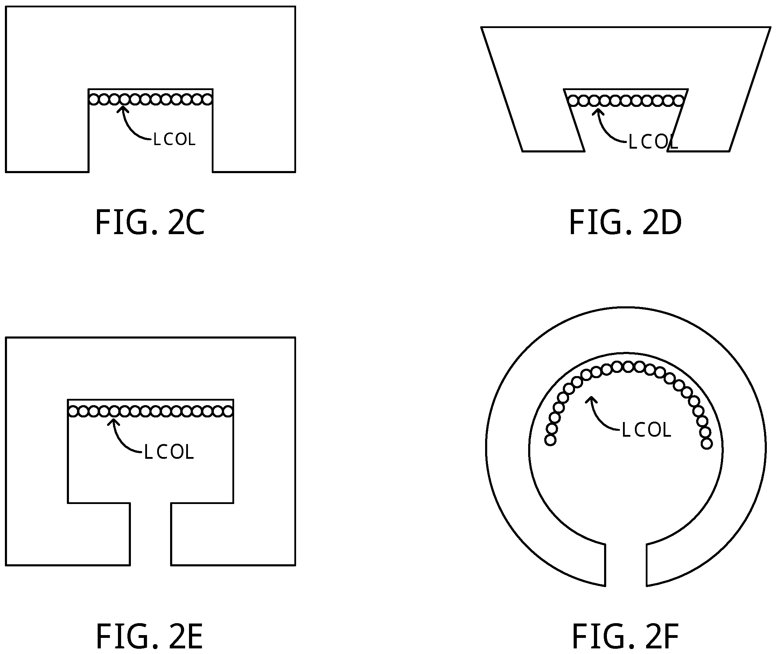

**FIG. 2A** is a schematic view depicting a cross-sectional structure of a core **COE**. The core **COE** has two side portions **SP** and a connecting portion **CP**. The core **COE** may be a ferromagnetic material, a ferrimagnetic soft magnetic material or a high saturation flux density material (e.g., an alloy containing Fe and Co) with a saturation density greater than 0.5 Tesla (T). When comparing a low saturation flux density material that is saturated to a high saturation flux density material that is under nearly-saturation operation, if the ratio of the saturation flux densities of the two materials is 3:2, then the magnetic resistance and the current required for generating the same magnitude magnetic field will be reduced by about 75%. The conductors of the coil **COL** may be copper wires, aluminum wires or other conductors (e.g., silver). The conductors of the coil **COL** pass through the groove **CG** of the magnetic core **COE** via the groove **CG**.

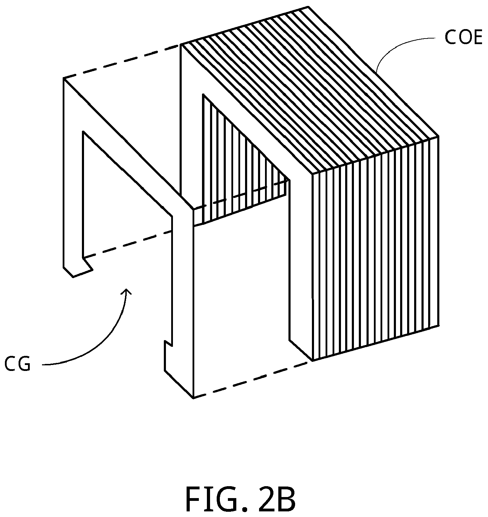

The common types of the core **COE** are: silicon steel sheet, ferrocobalt alloy, ferronickel alloy, magnetic powder core, amorphous, nanocrystalline, and so on. All of they usually have saturation densities greater than 0.5 T. The saturation densities of the silicon steel sheet and the ferronickel alloy are greater than 1.3 T, wherein the saturation density of the ferrocobalt alloy is even greater than 1.9 T. Although the aforesaid three alloys have good saturation density properties, the internal impedance thereof is relatively poor. Therefore, in order to enhance the impedance to resist the eddy current, the core **COE** is often structurally designed as sheets which are substantially perpendicular to the current direction, as shown in **FIG. 2B**. For example, the core **COE** may be formed of a plurality of iron core sheets. When the core **COE** is made of a high saturation flux density material, and the saturation density of the core **COE** is greater than 1.3 T, the thickness of each iron core sheet may be smaller than 1 mm. Besides, when the saturation density of the core **COE** is greater than 1.9 T, the thickness of each iron core sheet may be smaller than 0.5 mm. Furthermore, an insulating layer is presented between the sheets (i.e., between iron core sheets) to reduce the eddy current within the core **COE**.

Moreover, in other embodiments, the core **COE** may also be made of different materials (e.g., a magnetic powder core, an amorphous and a nanocrystalline) so that the core **COE** has built in an internal high-impedance structure, thereby reducing the eddy current within the core **COE** without using the sheet-shaped structure. Because there is a large airgap in the stimulator, so an airgap in the core is also allowable, or the core can be formed by a combination of a plurality of small cores without influencing the operation. The core saturation test adopts the Epstein frame measurement, and a magnetic field intensity of 1200A/m is selected.

The core **COE** may have various extended shapes, and the cross sections thereof are as shown in **FIG. 2C** to **FIG. 2F**. A conductor layer made of conductors with the same orientation at the lower part of the coil **COL** passes through the groove **CG**. When comparing to a plate-shaped core (as known as a groove with depth equal to 0mm), the grooved core (as known as an inverted U-shaped core) has relevance with the direct current (DC) loss, alternating current (AC) loss and thermal damage risk to the user. For DC losses: In a plate-shaped core, if a lower DC loss is desired, the thickness of the conductor layer would need to be increased in the plate-shaped core. Then, the distance between the core and the average current must increase. The-conductor would gradually lose its advantages provided by core, and become a conductor with air core property. Therefore, either a thicker conductor or a conductor closer to the core can solely be chosen. For a grooved core, due-to the two side extended portions **SP**, a shorter distance between core and conductor can still be provided while increasing the thickness of the conductor, and the magnetic field generated is still superposed at the lower part. Therefore, both benefits can be acquired at the same time.

Considering AC losses, there must be a fixed magnitude in the magnetic field for stimulation to be effective for the user. In the case where the width is much larger than the thickness in a conductor layer, because magnetic field only distributes downward, there is no great difference between the effects provided by the plate-shaped core or the groove core. For example, considering a conductor layer with a width of 3 cm and a thickness of 0.1 cm, providing a groove of 12 mm only reduces the loss by 20% at the frequency of 4kHz. However, due to the correlation with DC losses, the conductor layer cannot be made too thin, otherwise, the DC loss will be excessively large. Furthermore, for a thicker conductor layer, the magnetic field of the plate-shaped core would not only distribute downward, but also distribute leftward and rightward. Unlike the plate-shaped core, the magnetic field of the groove core would still distribute only downward due to the two side extended portions SP. Without the left and right portions exposed under the magnetic field, the proximity effect can be effectively reduced in the conductor layer, hence the AC loss is also reduced. For example, comparing to the plate-shaped core (as known as a groove with depth equal to 0mm), the power consumption is reduced by more than 45% at the frequency of 4kHz in a conductor layer with a width of 3 cm, a thickness of 6 mm, and a groove depth of 12 mm.

For thermal damage consideration: When the grooved core has two side portions **SP** extended beyond the lowest part of the conductor layer, because these distal ends of the two side portions **SP** serve as the proximate part of the stimulator and are-close to human body, such a configuration would make the conductor farther away from the human body, and the thermal damage to the human body by the coil **COL** is therefore reduced. Besides, the reduction of DC loss and AC loss will also reduce heat generation on their account.

The dimensional design of a core **COE** may enable the conductor to be at a distance from distal ends of the two side portions **SP** (e.g., the depth of the groove **CG** is 12 mm, the thickness of the conductor layer is 6 mm), so the thickness of the conductor layer is less than the depth. At least, the depth of the groove should cover the position of the average current of the lower conductor. For example, for a conductor layer of 6 mm, because the average current is at 3 mm, the depth of the groove should at least be larger than 3 mm in order to provide an obvious effect. The function gradually improves while the depth of the groove increases, and the function improvement is very obvious when the depth is about a half of the width of the opening of the core. When the depth of the groove is about half to one times the width of the groove, the function improves as the depth of the groove increases, but it is not obvious. When the depth of the groove is beyond one times the width of the groove, the function is less likely to improve as the depth of the groove increases. The shape of the core may change, so there is no absolute range theoretically. However, one suitable range of the depth of the groove that found in experiment is from 0.7 cm to 4 cm when considering weight and power consumption for portability.

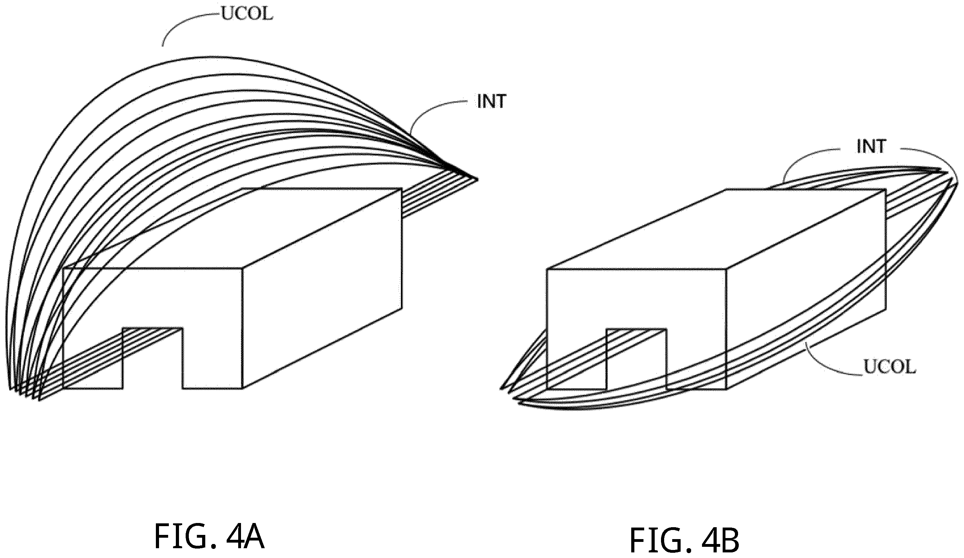

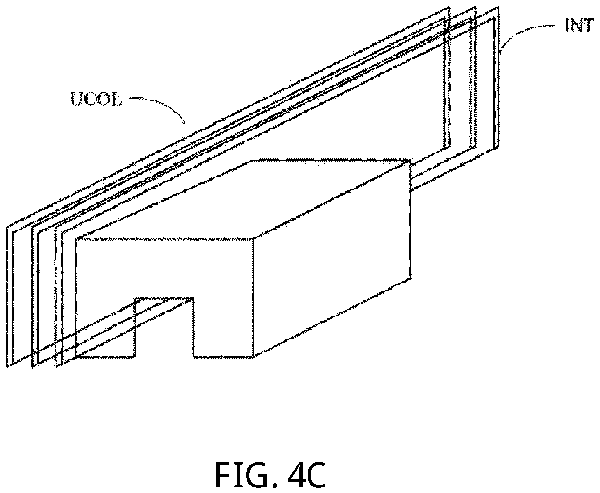

As compared to the lower part **LCOL**, the distance is greater between the average current of the upper part **UCOL** of the coil **COL** and the core **COE**, and the ratio is in the extent of more than two to one. The distance between the average current of the upper part **UCOL** of the coil **COL** and the core **COE** and the distance between the average current of the lower part **LCOL** of the coil **COL** and the core **COE** are calculated in the following way: (1) firstly, taking an current in space average vector of a single conductor; (2) then, taking an absolute value of the distance between the vector in space and the main body of the core; and (3) taking an average of the absolute values of the distances of all the conductors. The reason of using such a definition is as shown in **FIG. 4B**, for the upper part **UCOL** which is away from the core **COE** and has a suitable structure, the direct sum of vectors in space of all the conductors would fall in the core **COE**, and thus causes misjudgment. Adopting the aforesaid definition of (1) to (3), the distance can then comply with intuitive explanation that the upper part **UCOL** is farther away from the core **COE**.

For the upper coil in a grooved core, the upper part of the coil that is away from the core has some relevance-with the DC loss, the AC loss, the weight of the capacitor and the weight of the core. In the case of a conventional wound-type core, only one of the power consumption or the weight of the core can be prioritized. For example, if an upper coil of a larger cross-sectional area is adopted for better DC loss, the size of the groove core needs to be increased accordingly (because the cross-sectional area of the lower coil is the same as that of the upper coil, and the current of the lower coil needs to pass through the groove of the core), and the weight thereof also increases. When the upper coil is away from the core, the weight and the power consumption can be chosen at the same time (for the DC part, the cross-sectional area of the lower coil and the upper coil may be different, and the AC part is related to distribution in space). For example, in the case where the core and the lower coil does not change so that the function is not affected, adopting an upper coil of a larger cross-sectional area can reduce the DC loss and the AC loss, and adopting a plate-shaped, distributed upper coil can reduce the AC loss. Moreover, by keeping the upper part of the coil away from the core, the magnetic flux and inductance generated by the upper part of the coil with the core can be reduced. Saturation of the core can be prevented by reducing the magnetic flux generated by the core with the upper part of the coil, thereby reducing the thickness and weight of the core. The weight of the capacitor in the driver loop can be lowered by reducing the inductance generated by the core with the upper part of the coil. The weight of the capacitor and the weight of the core are the most significant parts among the components that are essential to the whole device.

The width of the connecting portion **CP** of the groove **CG,** the left-to-right width of the groove **CG**, including the lower core opening width and the maximum width where the conductor passes through, when taking size and weight factors into consideration, should all be in the extent of from 0.7 cm to 11.2 cm in order to coordinate with the stimulating depth. In order to prevent the saturation of the core, the thickness of the core **COE** should be in the extent from 0.4 cm and 4 cm. When the width of the opening of the core is further designed to be in the extent from 1.4 cm to 5.6 cm, and the thickness of the core **COE** is further designed to be in the extent from 0.7 cm to 2.8 cm, a better stimulating strength per unit weight can then be achieved. The lower part **LCOL** of the coil **COL** should be arranged from the position close to the inner edge of the groove **CG**, as shown in **FIG. 2C** to **FIG. 2F**; and if more coils are required, then the coils are superimposed downward. The width of the opening, the length and the height of the whole core should all be smaller than 11.2 cm to maintain the size and weight for portablity.

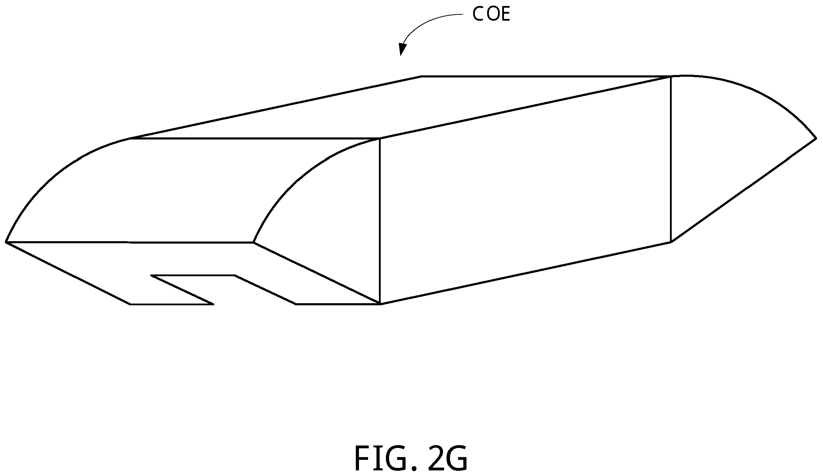

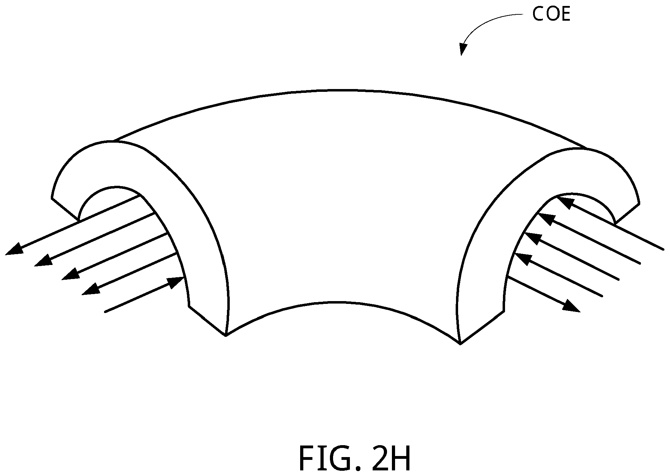

The leftward, rightward, downward or upward bending angles of the average current of the lower part **LCOL** of the coil **COL** at the entry and exit direction need to be within 60 degrees. For example, in **FIG. 2H**, the bending angle should be within 60 degrees when bending to the left. As shall be appreciated by those with ordinary skill of the art, according to the Biot-Savart law, the final magnetic field is determined by the integral sum of the current and the permeability of materials. The core **COE** may also bend and extend downward along the front end and the back end as shown in **FIG. 2G**, or bend toward one of the **SP** sides as shown in **FIG. 2H**. However, the difference of the magnetic field will not be too large according to the Biot-Savart law. Similarly, in **FIG. 2H**, the arrow indicates the current direction: the current direction on most of the conductors are the same, while the current direction in one conductor is opposite to the current direction of other conductors. According to the Biot-Savart law, the difference of the magnetic field caused by it will not be too large. Among the conductors of the lower part **LCOL** of the coil **COL**, the conductors with the current direction angle within 60° may be regarded as having the same direction, and they should represent more than 80% of all the conductors.

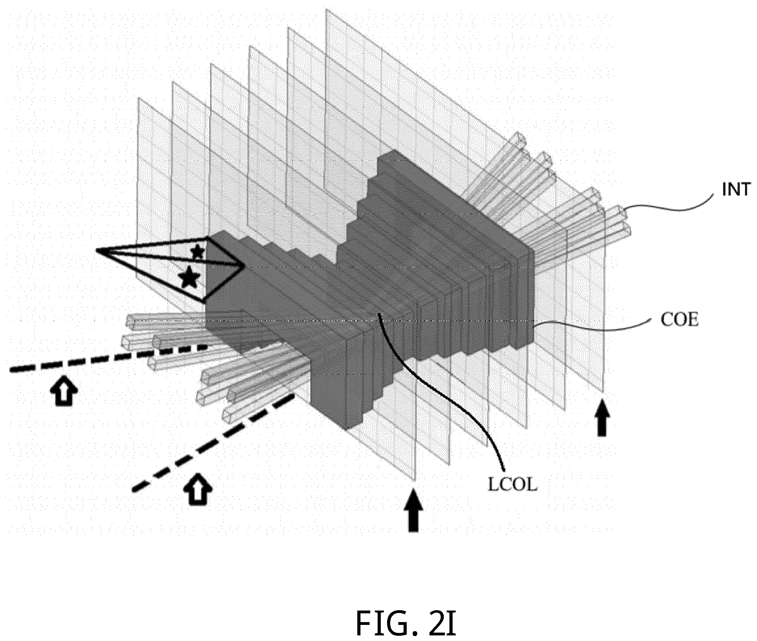

The required function of the stimulator can be achieved simply by some rectangular structures. However, in case if a core with an inordinate shape needs to be adopted, reference is made to **FIG. 2I** for the design principle. The rTMS apparatus will surely have a groove CG of the-grooved core **COE,** and a lower part **LCOL** of the coil **COL** that passes through the groove **CG,** and they should be closer to the user site than the connecting portion **CP** of the core **COE**, and the upper part **UCOL** of the coil **COL**. An average current direction is taken by the lower part **LCOL** of the coil **COL** with the part passing through the groove **CG** (i.e., the average current should pass through the groove of the core in the space), the average current direction is the normal vector of the tangential plane, and an entry tangential plane and an exit tangential plane can be defined for the core **COE**, as shown by the black arrow of **FIG. 2I**. The tangential planes are selected based on the principle of defining a core **COE** which is a bit shorter, while giving up the pieces that stand out from the rest, such as the star part of **FIG. 2I**. The reason to give up the pieces that stand out from the rest is that these pieces are incapable of-providing an improved function for the apparatus. The entry tangential plane and the exit tangential plane should be capable of defining the lower part **LCOL** of the coil **COL** that is initially selected, e.g., the part of eight conductors that is located between the two tangential planes shown in **FIG. 2I**. If there is a great difference, then the aforesaid steps are repeated and iterated to obtain the ideal tangential planes. The tangential plane is important because it can provide the following functions.

Firstly, it provides a judgement on the parameters mentioned in the above paragraphs: the width of the groove, the depth of the groove, the thickness of the core, the range of the upper part of the coil, the distance between the upper part of the coil and the core, and the distance between the lower part of the coil and the core. In case the stimulator is divided uniformly among these tangential planes between the entry tangential plane and the exit tangential plane, at least a 80% of sections should correspond to the parameters provided in the above paragraphs, while exact correspondence is not strictly required for every section, but the breadth should not deviate more than itself for at least 80% in these sections, and the core is conformed to a groove-shaped one.

Secondly, it can be used to determine the bending angle of the lower part of the coil: when the middle section between the entry tangential plane and the tangential plane going inward by 40% is defined as the entrance lower coil, and the middle section between the exit tangential plane and the tangential plane going inward by 40% is defined as the exit lower coil, then the average current of the entrance lower coil and that of the exit lower coil can be calculated respectively, from which the deviation angle can be calculated. Furthermore, in an inordinate core, because the lower coil should still be close to the core, the direction of different conductors within the lower part of the entrance coil may be different, as shown by the white arrow of **FIG. 2I**. However, 80% of the conductors should still have mutual offset angles less than 60 degrees, as it may be imagined that 80% of the conductors have directions in a cone with 60-degree pointing angle.

Thirdly, the two tangential planes may be used to distinguish: the upper part of the coil; the lower part of the coil, which is between the two tangent planes; and the part excluded, which can be regarded as interconnect part coil. The distance between the connecting coil and the core is not particularly limited and the connecting coil has a transitional property. The property of the part of the connecting coil that is close to the lower coil may not be similar to that of the lower coil because the distance between the part and the core is not as close as the distance between the lower coil and the core. However, the property of the part of the connecting coil that is close to the upper coil is similar to that of the upper coil because the distance between the part and the core is as far as the distance between the upper coil and the core. Fourthly, it is used to determine the length of the core, and the length of the core should range from 0.7 cm to 11.2 cm to maintain the portability. If the length is further designed to be between 1.4 cm and 5.6 cm, then a better stimulating strength can be achieved upon each unit energy.

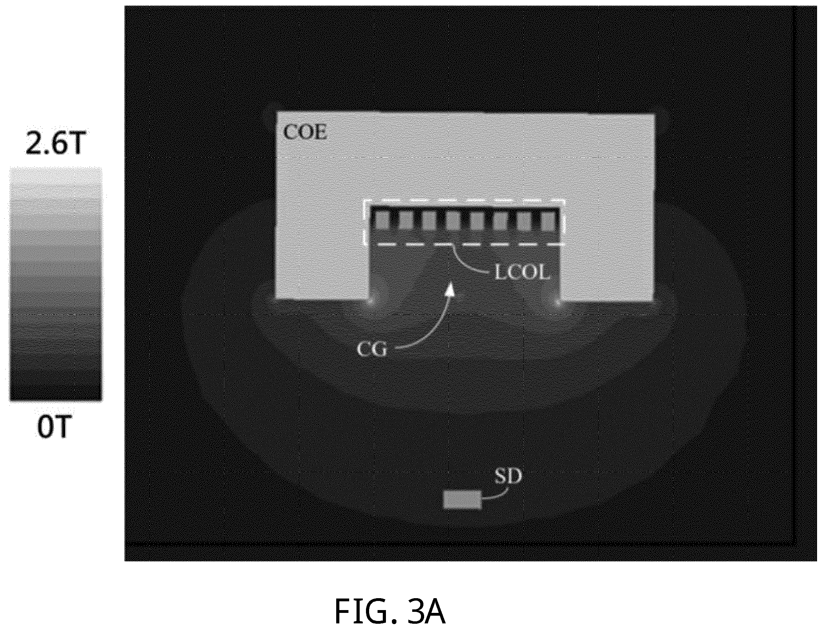

The width of the groove **CG** of the core **COE** needs to be selected according to the depth to be stimulated by the rTMS apparatus. For example, if the stimulating site is at the depth of 2.5 cm, as shown by a stimulating site **SD** of **FIG. 3A**, then the width of the lower opening of the groove **CG** shall be selected to be about 2.8 cm. Furthermore, the magnetic field of a conventional coil can distribute in six directions in an open space, i.e., upward, downward, leftward, rightward, frontward, and backward. If the design is modified into **FIG. 3A**, then only the downward distribution is left, i.e., only about one quarter of the energy is needed for magnetic field generation. Such a width selection may further create sufficient magnetic field gradient, and finally, it is the magnetic field gradient, rather than the absolute intensity, which is used to generate the stimulating electrical field. As a result, selecting the bent distributed magnetic field intensity will be more effective than selecting the straight distributed magnetic field intensity. The difference between some prior arts and this invention lies in that: the length of core groove and the conductor in some prior arts are greater than 11.2cm, and it would make the refluxing current more diversified in the stimulating part of the brain. However, the portable rTMS apparatus **1** selects the core groove and the conductor with lengths ranges from 0.7 cm to 11.2 cm, and it become not sure that the current will be more diversified in the brain. The objective of some prior arts is to provide a larger absolute magnetic field intensity at a depth (e.g., of 40 mm) within the brain so that a larger stimulating part and refluxing part are generated by the core, the portable rTMS apparatus **1** of the present invention, on the other hand, does not emphasize to increase the absolute magnetic field strength at a depth within the brain, but rather, it's main design emphasis is on "reducing the power consumption" and "reducing the weight".

Moreover, in **FIG. 3A**, if the stimulating site **SD** is at a depth of 2.5 cm, then a width of 2.8 cm of the groove **CG** may make the bending part located near the stimulating position. If an excessively large width (e.g., a conventional circular coil larger than 11.2 cm) is used, and the absolute intensity of the magnetic field is increased while the gradient is not increased, the design is therefore not being advantageous for the stimulating site **SD**. Moreover, the thickness of the core **COE** is selected mainly for preventing the saturation, and in practice, the thickness is greater than about 0.4 cm and smaller than about 4 cm. The length may also be designed based on the gradient principle, e.g., when the width of the lower opening of the groove **CG** is 2.8 cm, the length can also designed to be 2.8 cm, so that the gradient generated by the width and the length can be similar.

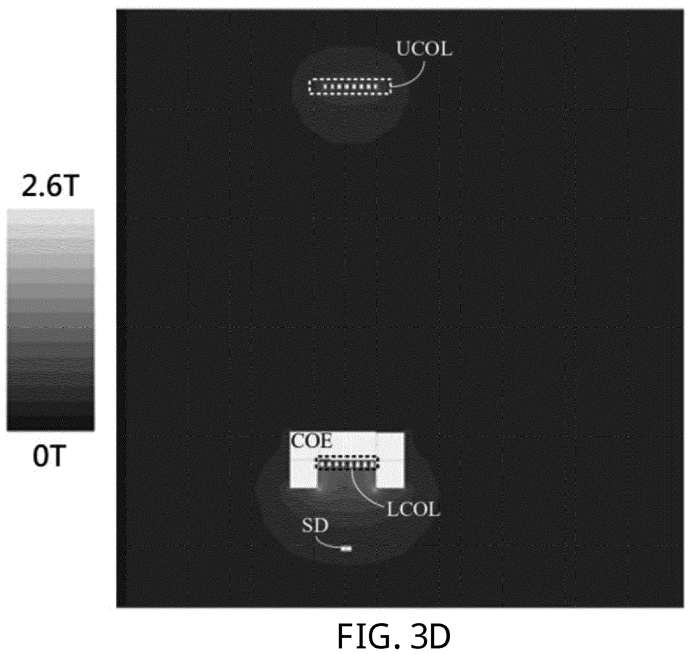

Distances between the upper part **UCOL** of the coil **COL** and the core **COE** are 0 cm, 6 cm and 12 cm in **FIG. 3B** to **FIG. 3D** **respectively.** Taking a model having a length of 5 cm in the direction normal to the plane, a frequency of 10 kHz and a power-on time of 0.2% as an example, if the way of winding of **FIG. 3B** is used to generate a magnetic field with a maximum value of 0.2 T at the depth of 2 cm, it can be calculated that the maximum energy storage in the total inductor is 14 J, and the AC loss is 8.14 W. If the upper part **UCOL** of the coil **COL** is moved farther away from the core by 6 cm, as shown in **FIG. 3C**, then the maximum energy storage is changed to 12 J, and the power consumption is changed to 5.44 W. In other words, as the upper part **UCOL** of the coil **COL** is moving away from the core **COE**, then under the same stimulating strength, the total energy storage can be changed to 86%, and the power consumption can be changed to 70%. If the distance between the upper part **UCOL** of the coil **COL** and the core **COE** is further increased to 12 cm, as shown in **FIG. 3D**, it can be observed that the power consumption and the energy storage are not changed anymore.

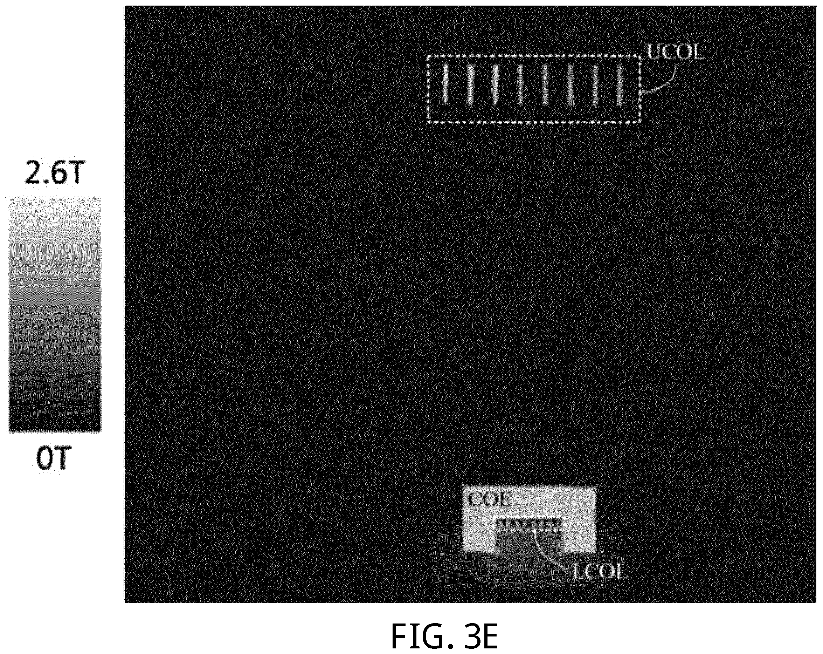

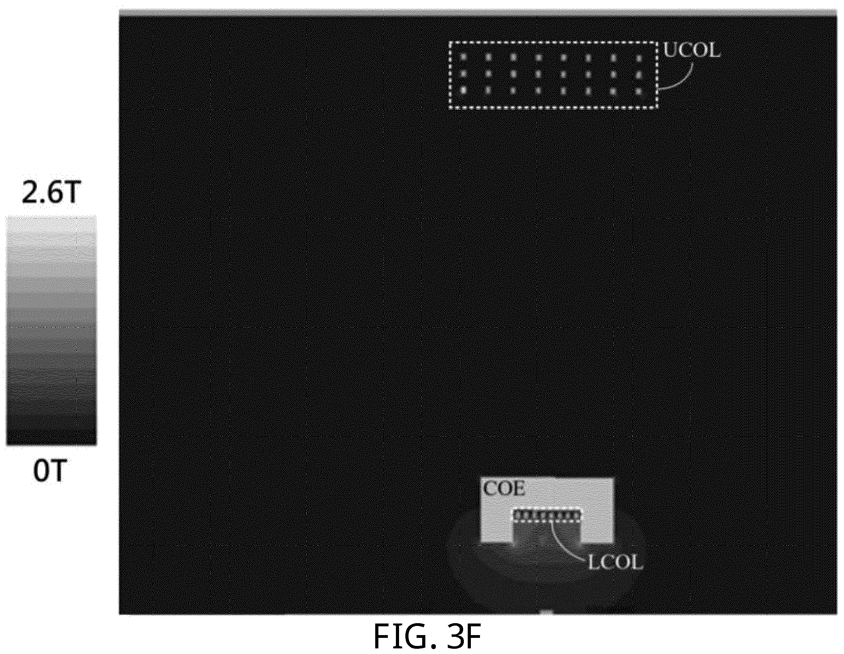

Other than keeping the upper part **UCOL** of the coil **COL** and the core **COE** away, the shape of the upper part **UCOL** of the coil **COL** also influences the power consumption and the energy storage. As shown in **FIG. 3E**, the upper part **UCOL** of the coil **COL** is formed of eight conductor sheets having a total width of 5.4 cm and a total height of 1.2 cm, and thus the total energy storage can be changed to 11 J, and the total power consumption can be changed to 4.1 W. In other words, if the upper part **UCOL** of the coil **COL** is far away from the core **COE** and adopts conductors of larger space distribution, then under the same stimulating strength, the total energy storage can be reduced to 79% and the power consumption can be reduced to 50%. This phenomenon is due to the fact that the AC resistance is mainly generated by the magnetic field, and if the same current is distributed in a larger space, then the power consumption can be further reduced (e.g., by adopting a wider conductor sheet or thicker conductors). Because the weight of the conductor having a larger cross-section area is greater, the design shown in **FIG. 3F** may be adopted by replacing the 8 conductor sheets of **FIG. 3E** with 24 conductor of **FIG. 3F** on the upper part **UCOL** of the coil **COL**, while the lower part **LCOL** of the coil **COL** remain at 8 conductors. That is, a conductor of the lower part **LCOL** of the coil **COL** extends out of the groove and is divided into 3 conductors and the 3 conductors are again incorporated into a conductor when it is winded back to the lower part **LCOL** of the coil **COL**. In **FIG. 3F**, the total width of the upper part **UCOL** of the coil **COL** in the space is 5.4 cm, the total height thereof is 1.2 cm and is still the same as that of **FIG. 3E**, and thus the total energy storage can be changed to 11 J, and the total power consumption is changed to 4.14 W. Moreover, another advantage of enlarging the equivalent cross-sectional area of the upper part **UCOL** of the coil **COL** is to reduce the leakage inductance, and to improve the coupling situation.

In another aspect, when the conductors of the lower part **LCOL** of the coil **COL** extend outward and are connected into a loop outside the core **COE** via the connecting part **INT** and the upper part **UCOL** of the coil **COL**, the number of the conductors of the lower part **LCOL** may be less than the number of the conductors of the upper part **UCOL** (e.g., be a half of the number of the conductors of the upper part **UCOL**). That is, a first number of conductors of the lower part **LCOL** of the coil **COL** is less than a second number of conductors of the upper part **UCOL** of the coil **COL**. Or, the multiple conductors in upper part **UCOL** can be substituted with a large cross-sectional area conductor instead. The present invention when under such configuration, not only being capable to reduce the DC resistance, but also reduce the proximity effect and the AC resistance further.

In other embodiments, a conductor spacing of the upper part **UCOL** of the coil **COL** is larger than a conductor spacing of the lower part **LCOL** so that a total conductor spacing of the upper part **UCOL** is larger than a total conductor spacing of the lower part **LCOL** of the coil **COL** to reduce the AC impedance.

In other embodiments, the upper part **UCOL** of the coil **COL** may be presented as distributed or branched, as shown in **FIG. 4A** to **FIG. 4C**. The distribution of the upper part **UCOL** of the coil **COL** may further reduce the summation of the magnetic field, thereby reducing the proximity effect and the AC impedance. The branched upper part **UCOL** of the coil **COL** may further reduce the total resistance. The thermal energy generated by the lower part **LCOL** of the coil **COL** can be directed to the upper part **UCOL** of the coil **COL** via conductors. The upper part **UCOL** of the coil **COL** has a very large contacting area with air, so it can effectively dissipate heat. It shall be appreciated that, **FIG. 4A** to **FIG. 4C** are only used to illustrate the structure of the coil **COL**, so the upper fastening component **UFC** and the lower fastening component **LFC** are not depicted therein.

Moreover, in other embodiments, the upper part **UCOL** of the coil **COL** may use conductors of a larger area, i.e., a conductor width of the upper part **UCOL** is larger than a conductor width of the lower part **LCOL** so that the cross-sectional area of the conductors of the upper part **UCOL** is larger than the cross-sectional area of the conductors of the lower part **LCOL** of the coil **COL** to reduce the AC impedance.

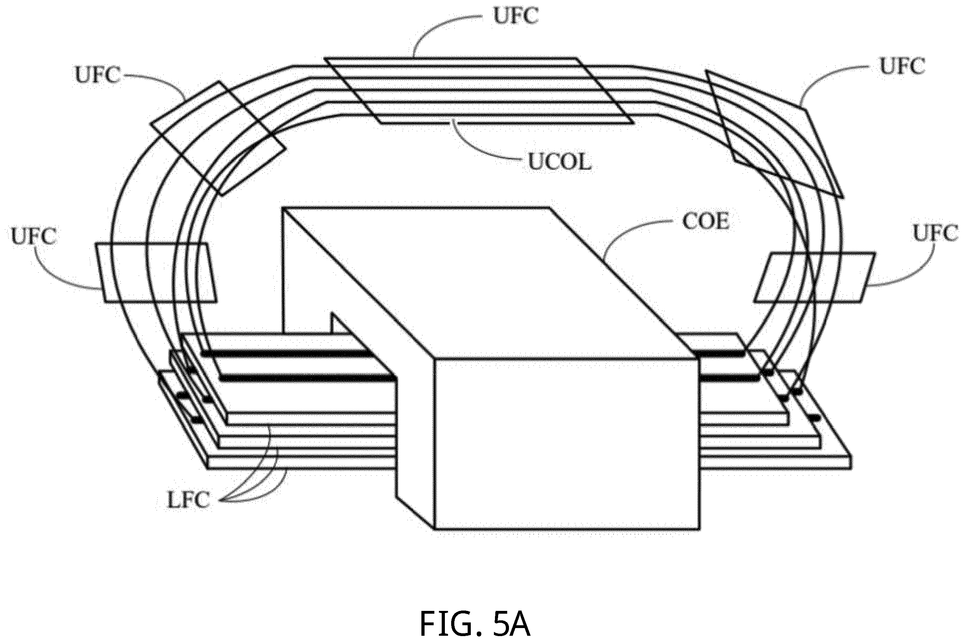

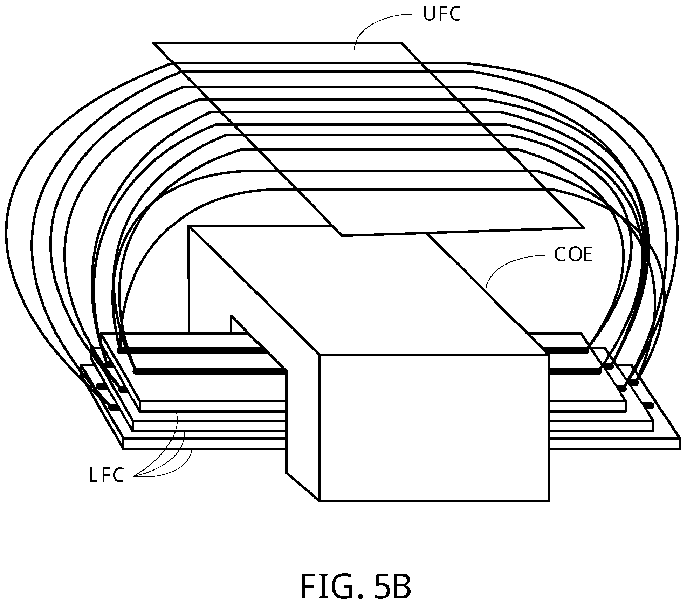

Referring to **FIG. 5A** to **FIG. 5B**, the upper fastening component **UFC** and the lower fastening component **LFC** are all insulators (e.g., plastic materials, ceramic materials) and are respectively used for fastening the upper part **UCOL**, the lower part **LCOL** and the connecting part **INT** of the coil **COL**, thereby preventing the conductors from contacting with each other due to intertwined coil. The number of conductors of the upper part **UCOL** of the coil **COL** may be greater than the number of conductors of the lower part **LCOL**, or sheet-shaped conductors or conductors of a larger diameter are adopted as the conductors, as a result, a larger area is required to serve as the upper fastening component **UFC** for maintaining the structure of the upper part **UCOL** of the coil **COL**, as shown in **FIG. 5B**.

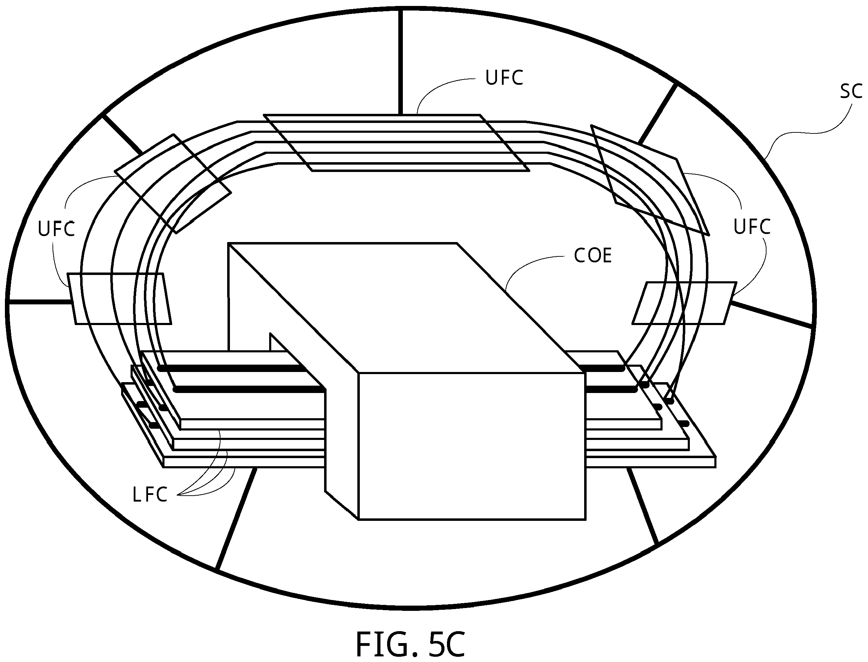

In other embodiments, the inductor **L** further includes a supporting component **SC**, the supporting component **SC** is also an insulator and is winded around the inductor **L**, and the supporting component **SC** contacts with the upper fastening component **UFC** and the lower fastening component **LFC** to enhance the effect of maintaining the structure of the upper part **UCOL** and the lower part **LCOL** of the coil **COL** by the upper fastening component **UFC** and the lower fastening component **LFC**, as shown in **FIG. 5C**.

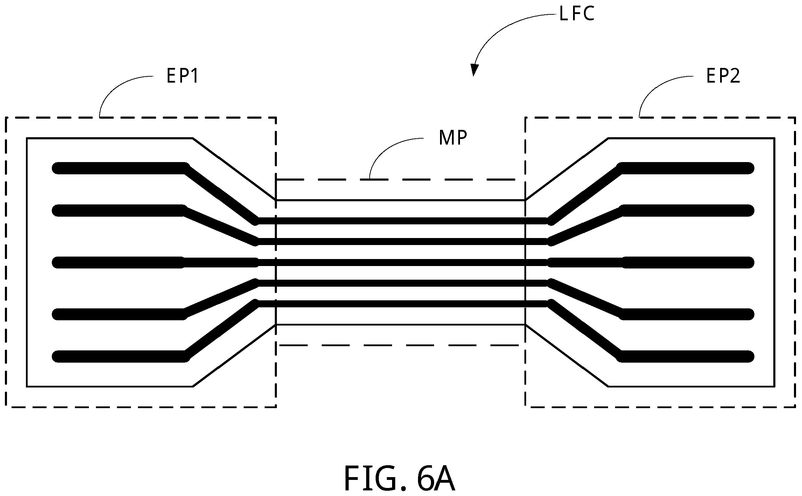

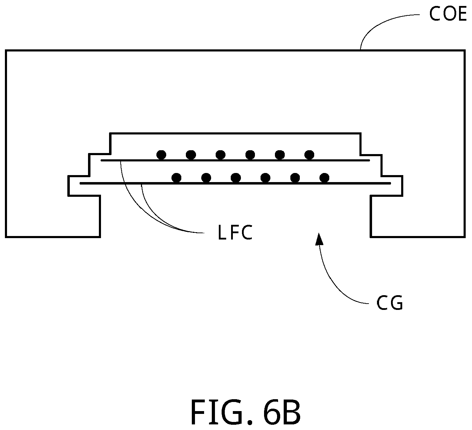

**FIG. 6A** depicts a schematic view of the lower fastening component **LFC**. The lower fastening component **LFC** includes a plurality of ceramic substrates, and each of the ceramic substrates has a middle part **MP**, a first end **EP1** and a second end **EP2**. The first end **EP1** and the second end **EP2** are connected via the middle part **MP**. These ceramic substrates are stacked into a layered structure, and the connecting portion **CP** of the core **COE** shields the middle parts **MP** of the ceramic substrates. The lower fastening component **LFC** may be fastened within the groove of the core **COE** in a stepped or cogged manner, as shown in **FIG. 6B**.

Moreover, for any two adjacent ceramic substrates among the ceramic substrates **CS**, a size of an upper ceramic substrate is smaller than that of a lower ceramic substrate so that a ladder shape is formed respectively by the first ends **EP1** and the second ends **EP2** (but not limited thereto). For example, as shown in **FIG. 1**, in the lower fastening components **LFC**, the length of a second ceramic substrate is larger than the length of a first ceramic substrate, and the length of a third ceramic substrate is larger than the length of the second ceramic substrate. In this case, the length of the conductors on the first end **EP1** and the second end of the upper ceramic substrate will be smaller than the length of the conductors on the first end **EP1** and the second end of the lower ceramic substrate.

It shall be appreciated that, the shape of the lower fastening component **LFC** may vary according to requirements of circuit arrangement. For example, the lower fastening component **LFC** may be a ceramic substrate of a rectangular shape as shown in **FIG. 1**, or a dumbbell-shaped ceramic substrate as shown in **FIG. 6A**. Moreover, aluminium oxide, aluminium nitride, silicon nitride, silicon carbide and/or other thermal-conductive but not electrical-conductive ceramic materials may be used as the ingredients of the ceramic substrates. How to design the appearance and shape of the ceramic substrates and determine the material of the ceramic substrate according to different requirements of circuit arrangement shall be appreciated by those of ordinary skill in the art, and thus will not be further described herein.

The heat-dissipating speed of the inductor **L** can be improved by disposing the lower part **LCOL** of the coil **COL** on the ceramic substrate, so the inductor **L** will not be destroyed when the current on the coil **COL** is concentrated at a position in the space (e.g., the middle part **MP** of the ceramic substrate). A conductor width of the coil **COL** that is disposed at the first end **EP1** and the second end **EP2** may be larger than a conductor width of the coil **COL** that is disposed at the middle part **MP**. Moreover, the ceramic substrate and the lower part **LCOL** of the coil **COL** may be directly joined via a specific process (e.g., a thin film process, a thick film process, a direct bonding copper technology, a direct plating copper technology and an active metal hard welding technology or the like).

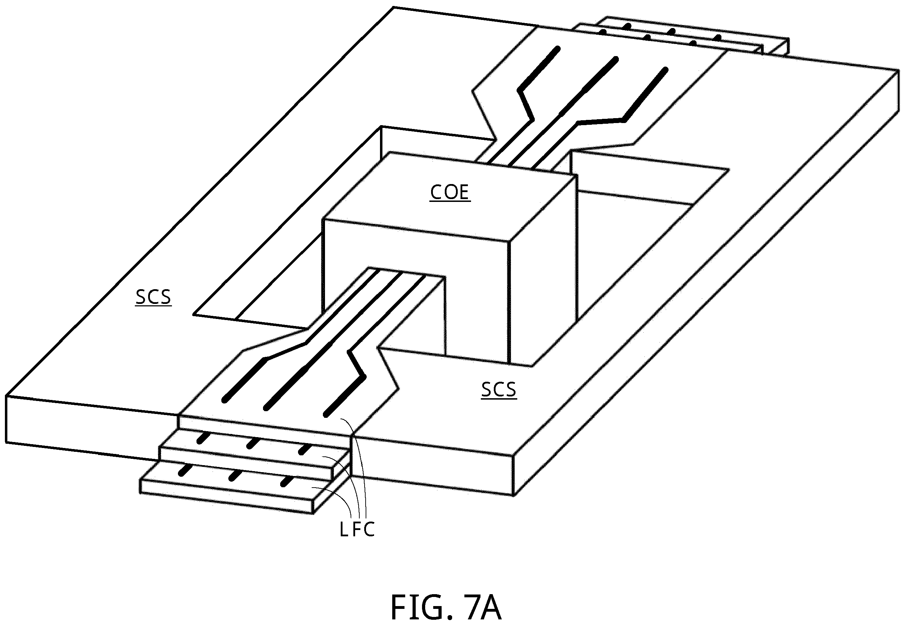

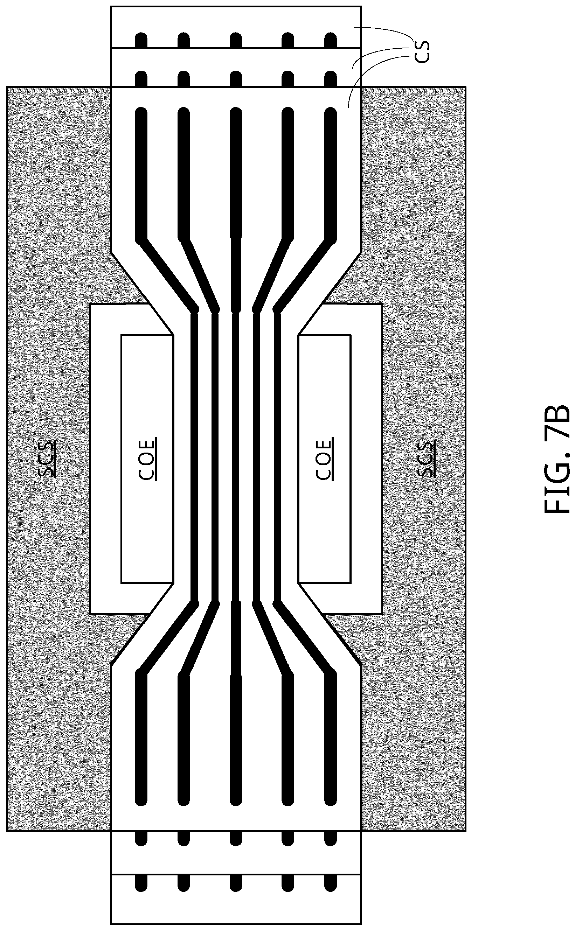

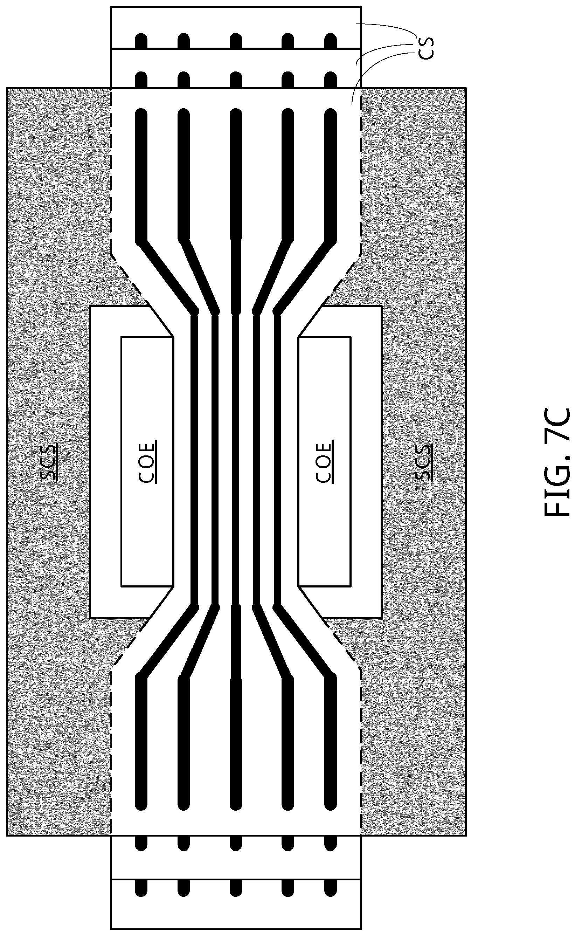

In other embodiments, the inductor **L** may further include a plurality of extended ceramic substrates **SCS** which, as shown in **FIG. 7A**, may be manufactured to have the same height as these lower fastening components **LFC** after being stacked and contact with the lower fastening components **LFC**. The extended ceramic substrate **SCS** may also be manufactured to contact with the lower fastening components **LFC** respectively along the two side portions **SP** of the core **COE**, as shown in **FIG. 7B**. Moreover, when the lower fastening components **LFC** are ceramic substrates, these extended ceramic substrates **SCS** may be further designed to be formed integrally with the ceramic substrates, as shown in **FIG. 7C**. Moreover, in another embodiment, the extended ceramic substrates **SCS** may be manufactured to have the same thickness with the lower fastening components **LFC** or become a part of the lower fastening components **LFC** (i.e., formed integrally with the lower fastening components **LFC**), and stacked sequentially along with the lower fastening components **LFC**.

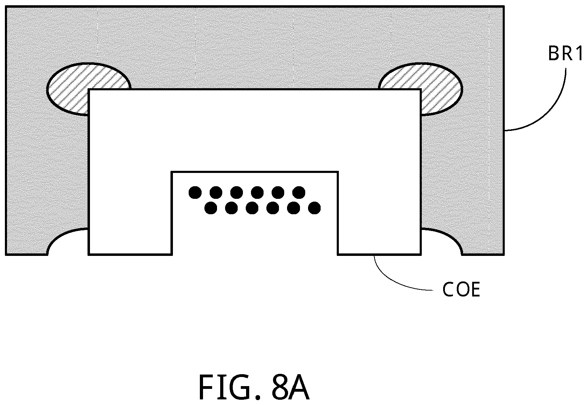

In other embodiments, the inductor **L** further includes an upper blocking component **BR1**, and the core **COE** of the inductor **L** is at least partly covered by the upper blocking component **BR1**, as shown by the grey part of **FIG. 8A**. The upper blocking component **BR1** may reduce the noise generated by the inductor **L** during the use thereof. The upper blocking component **BR1** is made of a material of high hardness, e.g., ceramic, silicon carbide, aluminum oxide or the like. The material of high hardness is a substance of which the young's modulus is at least greater than 50 GPa (gigapascal). Moreover, the upper blocking component **BR1** further comprises part cavities (i.e., the part indicated by slash lines in **FIG. 8A**), and the cavities may prevent the breakage of the upper blocking component **BR1** due to the vibration of the inductor **L** generated during the operation thereof.

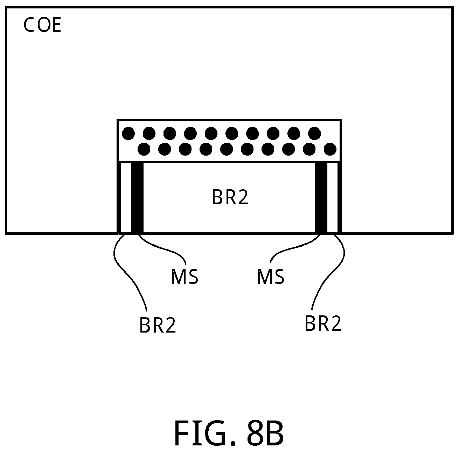

In other embodiments, the inductor **L** further includes a lower blocking component **BR2** and a magnetostrictive material **MS**, and the lower blocking component **BR2** and the magnetostrictive material **MS** are disposed at the inner sides of two side portions of the core **COE**, as shown in **FIG. 8B**. The magnetostrictive material is a substance with a magnetostriction ratio greater than 100 ppm. Specifically, deformations would appear during the operation of the rTMS apparatus **1** due to the Lorent force, the reluctance force and the magnetostriction, and they would cause the core **COE** and the coil **COL** to vibrate and generate noise. The coil **COL** is mainly influenced by the Lorentz force, while the core **COE** is mainly influenced by deformations caused by the reluctance force and magnetostriction.

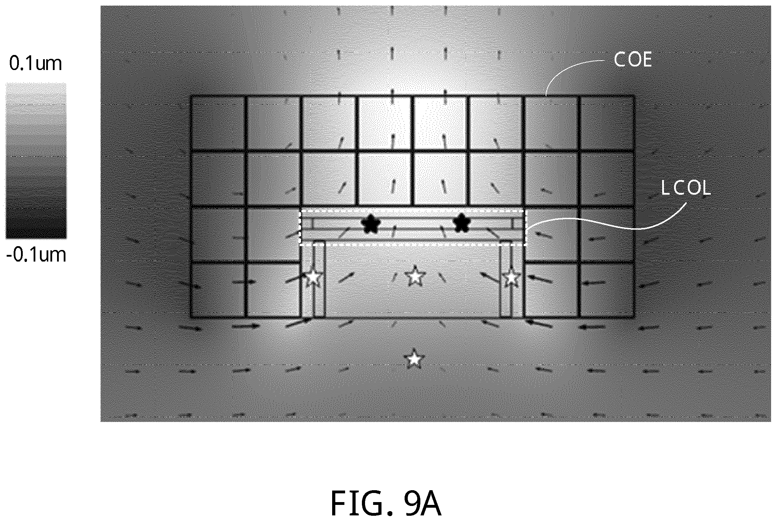

For the magnetic field intensity having a value of 0.2 T at the depth of 2 cm, when only the reluctance force to the core **COE** and the Lorentz force to the coil **COL** are taken into consideration and the magnetostriction is not taken into consideration, the directions of the deformations are as shown by the directions of the arrows in **FIG. 9A**. Taking the case where the young's modulus of the core **COE** is equal to 200 Gpa and the ambient air is 2 GPa for calculation, the upward deformation from the central portion (i.e., the connecting part **CP**) of the core **COE** is 0.14 um, and the inward deformations from the two side portions **SP** are 0.2 um. It shall be appreciated that, the young's modulus of the ambient air is set to 1% of the young's modulus of the core **COE** for preventing the calculation result being influenced; hence, it is not the real hardness.

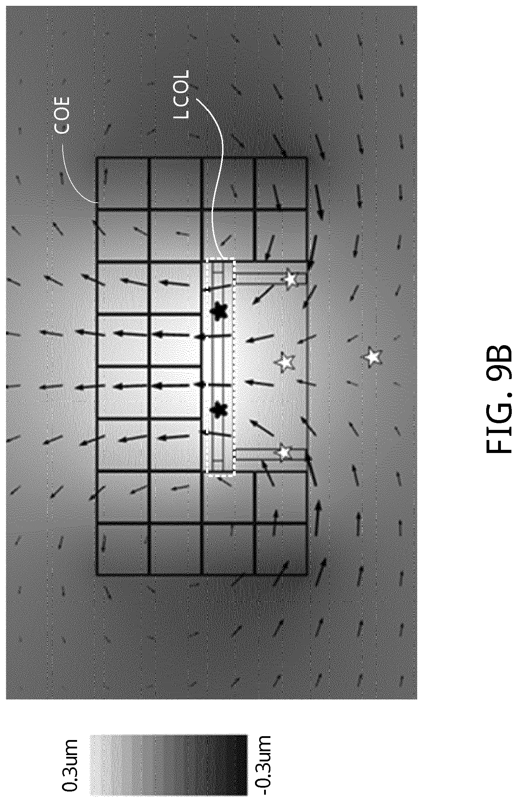

In the case where the reluctance force of the core **COE**, the Lorentz force of the coil **COL** and the magnetostriction are taken into consideration at the same time, the directions of the deformations are as shown by the directions of the arrows in **FIG. 9B**. If the lambda of the core **COE** is set to be 3e-5, the saturation magnetization is set to be 2e6, then it can be observed that the upward deformation from the central portion of the core **COE** is 0.22 um, the inward deformations from the two side portions **SP** are still 0.2 um, and the direction of the deformation is different from that of **FIG. 9A**. In other words, the deformation caused by part reluctance force and the deformation caused by the force of magnetostriction may be added or canceled with each other because the directions thereof may not be the same, thereby causing different deformations in shape.

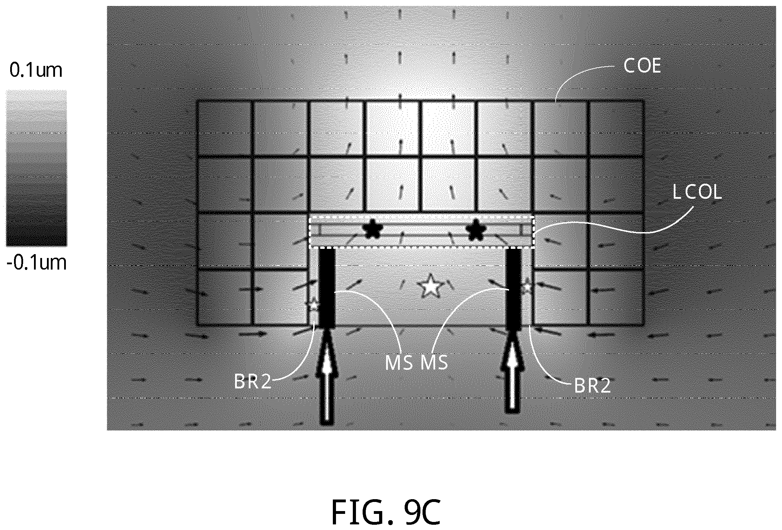

In other embodiments, the inductor **L** further includes a lower blocking component **BR2**, and inner sides of the two side portions **SP** of the core **COE** are connected with the lower blocking component **BR2**, as shown in **FIG. 9C**, and the lower blocking component **BR2** can reduce the noise generated by the inductor **L** during the operation. The lower blocking component **BR2** is made of a material of high hardness, e.g., ceramic, silicon carbide, aluminum oxide or the like. The lower blocking component **BR2** may include a magnetostrictive material **MS** other than the core **COE**. As shown in **FIG. 9C**, the magnetostrictive material **MS** is added at the inner sides of the two side portions **SP** of the core **COE**, the lambda thereof is 2e-3, the saturation magnetization is 1e6, the young's modulus is 30 Gpa, the upward deformation from the central portion of the core **COE** can be further set to be 0.02 um, and the inward deformations of the two side portions **SP** are 0.07 um.

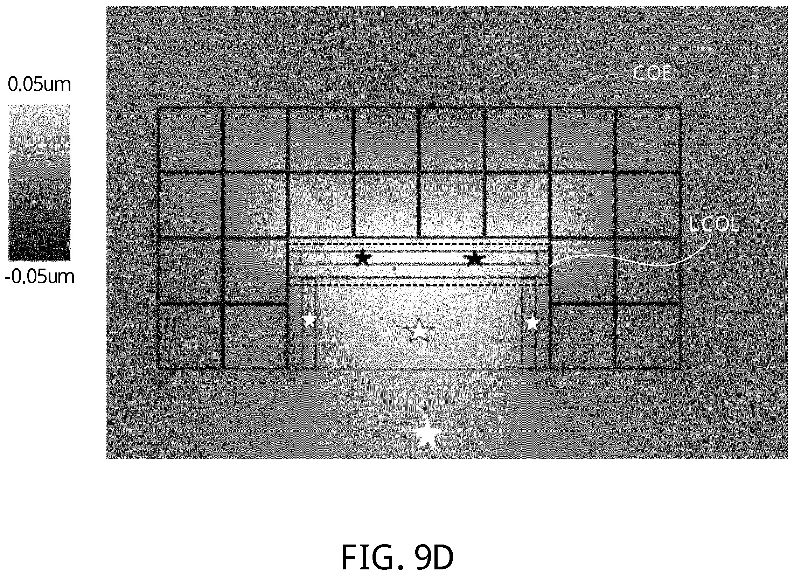

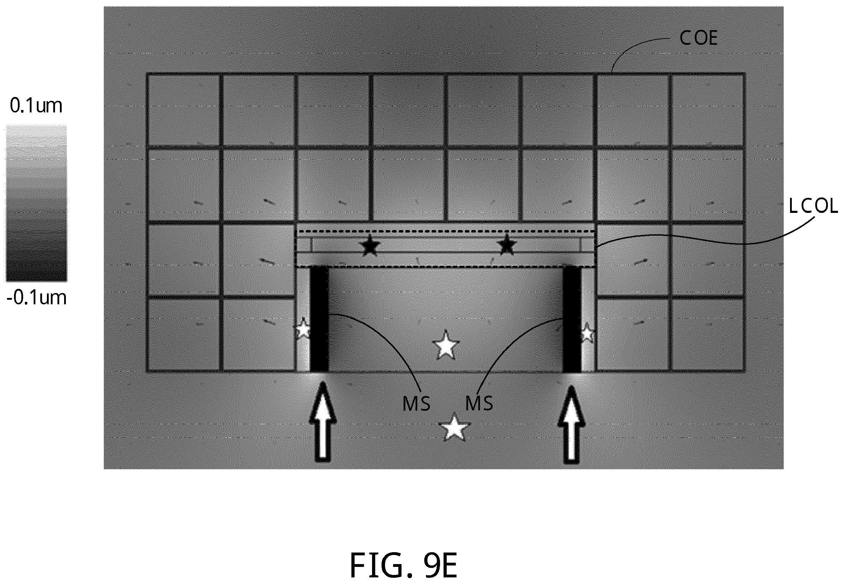

In other embodiments, the strength of the surrounding structure may be enhanced to reduce the vibration. For example, if the young's modulus of the upper blocking component **BR1** and the lower blocking component **BR2** is changed into 200 Gpa, then the upward deformation from the central portion of the core **COE** is 0.006 um, and the inward deformations of the two side portions **SP** are 0.013 um, as shown in **FIG. 9D**. Moreover, for the case where the magnetostrictive material **MS** is added, if the young's modulus of the upper blocking component **BR1** and the lower blocking component **BR2** is changed into 200 Gpa, then the upward deformation from the central portion is 0.018 um, and the inward deformations of the two side portions **SP** are 0.03 um, as shown in **FIG. 9E**. The thickness of the upper blocking component **BR1** should range from 2 mm to 20 mm to match the thickness of the core.

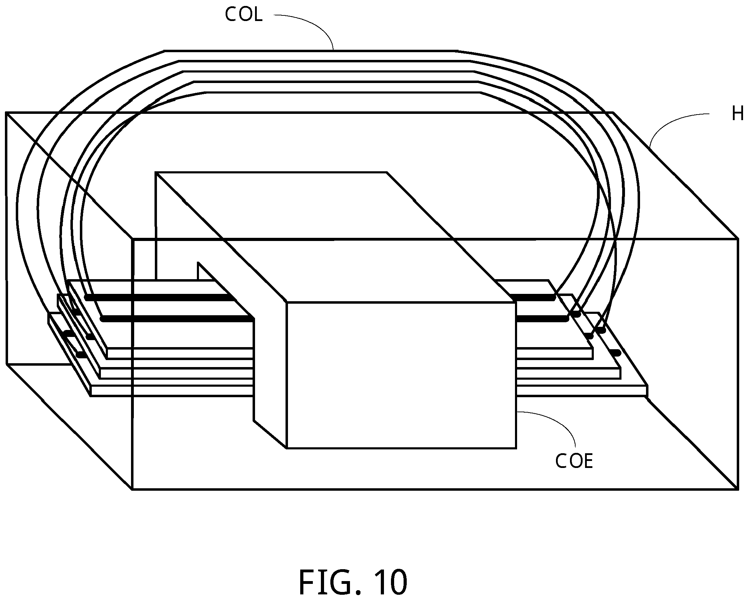

In other embodiments, the rTMS apparatus **1** further comprises a housing **H**, and the inductor **L** is disposed within the housing **H**, as shown in **FIG. 10**. The material of the housing **H** has high acoustic impedance, and the housing **H** at least covers main vibration sources such as the core **COE** and the lower part **LCOL** of the coil **COL** in the space, thereby reducing the transmission of noise. At least one layer of material having low acoustic impedance (e.g., air, porous sound absorbing materials or the like) is used between the housing **H** and the inductor **L** to increase the reflection. The core **COE**, the lower part **LCOL** of the coil **COL** and a part of the connecting portion **INT** are located within the housing **H**. A spring with a specific damping and elastic coefficient used between the inductor **L** and the housing **H** may make the self-vibration frequency of the inductor **L** which is generated from the current, and the vibration frequency of combined inductor **L** and the housing **H,** being separated. Therefore, the sound wave generated by the housing **H** is reduced. Moreover, the housing **H** and the inductor **L** are only connected via the specific spring and damp without other contact. Therefore, when a part of the upper part **UCOL** of the coil **COL** is disposed outside the housing **H**, the connection of the connecting portion **INT** of the coil **COL** and the housing **H** should be taken into consideration when considering elastic coefficient and damping. Moreover, if a porous sound absorbing material is filled between the housing **H** and the core **COE**, then the porous sound absorbing material also needs to be taken into consideration.

Moreover, the sound wave generated by the core **COE** and the conductor is reflected back into the cavity (i.e., the space between the inductor **L** and the housing **H**) when it encounters high acoustic impedance, and is oscillated in the cavity and finally absorbed. The spacing between the inductor **L** and the housing **H** may be designed according to the one-quarter-wavelength principle. For example, the wavelength corresponding to the frequency of 10 kHz is about 3.4 cm, so adopting a cavity having a one-quarter wavelength of 0.85 cm and filling the porous sound absorbing material or other sound absorbing materials can help to transform the sound wave oscillated in the cavity into thermal energy and make it finally being absorbed. Additionally, in other embodiments, the upper part **UCOL** of the coil **COL** may also be completely disposed within the housing **H**. The housing **H** and the core **COE** are separated by at least a layer of substance having low acoustic impedance therebetween. The housing **H** and the core **COE** are joined by a spring. An elastic coefficient of the spring ranges from 0.001 mm/N to 0.1 mm/N. A thickness of the housing **H** ranges from 0.5 mm to 1 cm. A thickness of the substance having low acoustic impedance ranges from 0.4 cm to 8 cm. The substance having low acoustic impedance is defined as a substance below 0.01 MPa*s/m³, and a substance having high acoustic impedance is defined as a substance ranging from 1 to 100 MPa*s/m³. The implementation of this aspect shall be appreciated by those of ordinary skill in the art, and thus will not be further described herein.

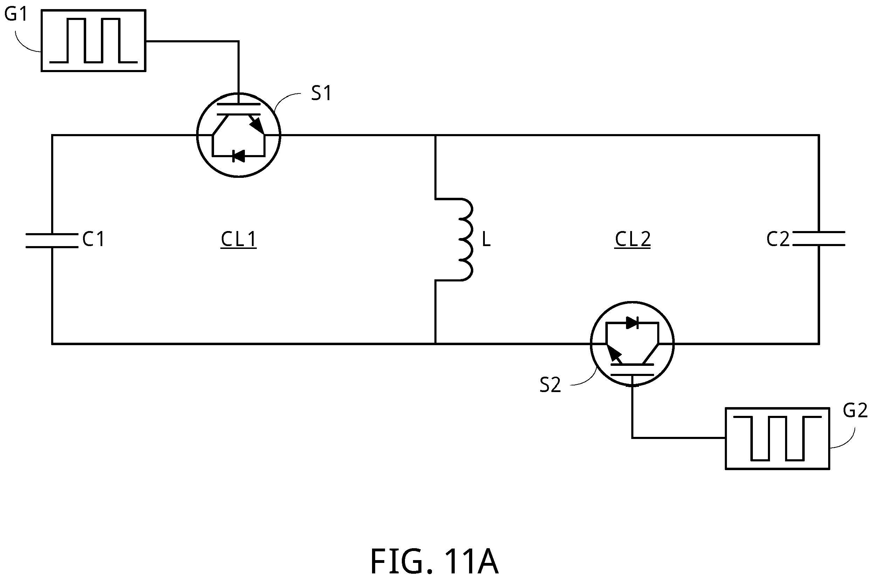

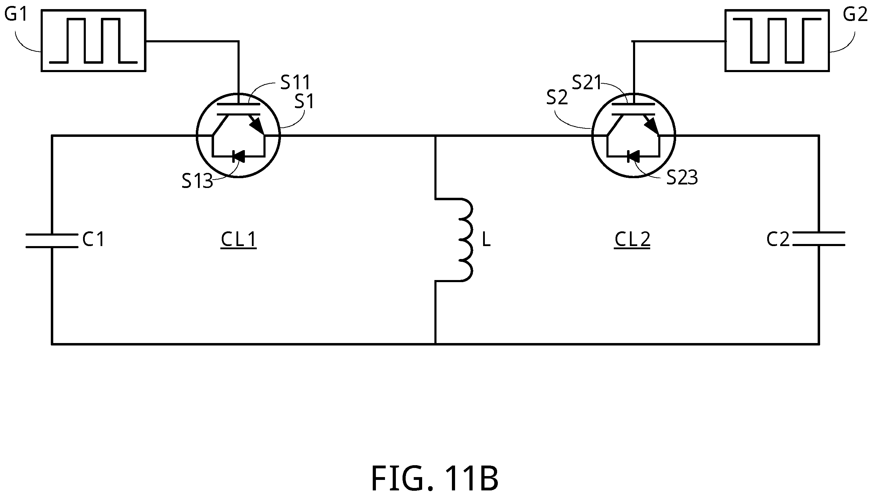

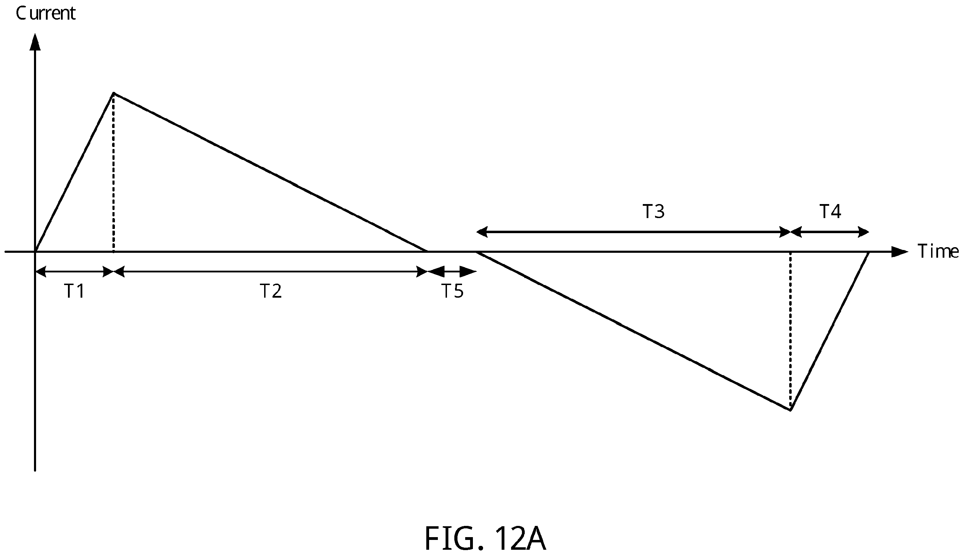

A second embodiment of the present invention is as shown in **FIG. 11A** to **FIG**. **11B**, **FIG. 12A** to **FIG. 12E**, **FIG. 13** to **FIG. 14**, **FIG. 15A** to **FIG. 15C**, **FIG. 16** and **FIG. 17A**, and it is an extension of the first embodiment. **FIG. 11A** and **FIG. 11B** are schematic views depicting the circuits of the portable rTMS apparatus **1** of the present invention. The driver circuit **DRC** comprises a first capacitor **C1**, a second capacitor **C2**, a first switch with freewheeling diode **S1**, a second switch with freewheeling diode **S2**, a first pulse generator **G1** and a second pulse generator **G2**.

The first energy storage capacitor **C1**, the first switch with freewheeling diode **S1** and the inductor **L** are connected in series to form a first driver circuit loop **CL1**. The second energy storage capacitor **C2**, the second switch with freewheeling diode **S2** and the inductor **L** are connected in series to form a second driver circuit loop **CL2**. The aforesaid circuit is connected via a half-bridge primary side. It shall be appreciated that, the schematic views of the circuits in **FIG. 11A** to **FIG. 11B** are not intended to limit the sequence of the first energy storage capacitor **C1** and the first switch with freewheeling diode **S1** in the first driver circuit loop **CL1** and the sequence of the second energy storage capacitor **C2** and the second switch with freewheeling diode **S2** in the second driver circuit loop **CL2**. In practical application, the provider or the manufacturer of the portable rTMS apparatus **1** may decide the order in which the capacitors and the switches are connected in series according to different requirements.

The first driver circuit loop **CL1** comprises the first pulse generator **G1** that is coupled to the first switch with freewheeling diode **S1** and generating a first pulse signal to control the first switch with freewheeling diode **S1**. The second pulse generator **G2** is coupled to the second switch with freewheeling diode **S2** and generating a second pulse signal to control the second switch with freewheeling diode **S2**. The first switch with freewheeling diode **S1** has a controllable switch **S11** and a diode **S13** and the second switch with freewheeling diode **S2** has a controllable switch **S21** and a diode **S23**.

The switch with freewheeling diode is defined as an electrical network that is equivalent to a controllable switch connected in parallel with a reverse diode on the scale of circuit in household power suppliers. The switch with freewheeling diode can turn on bidirectional current when a control signal is activated, and it can turn on the reverse current or block the forward voltage when the control signal is deactivated. Because embodiments using the switch with freewheeling diode all have two loops causing different magnetic flux directions, the diode of the second driver circuit loop **CL2** is immediately turned on when the first driver circuit loop **CL1** is turned off, and thus it is called a freewheeling diode. The switches with freewheeling diode mentioned in the subsequent embodiments are all defined in the same way.

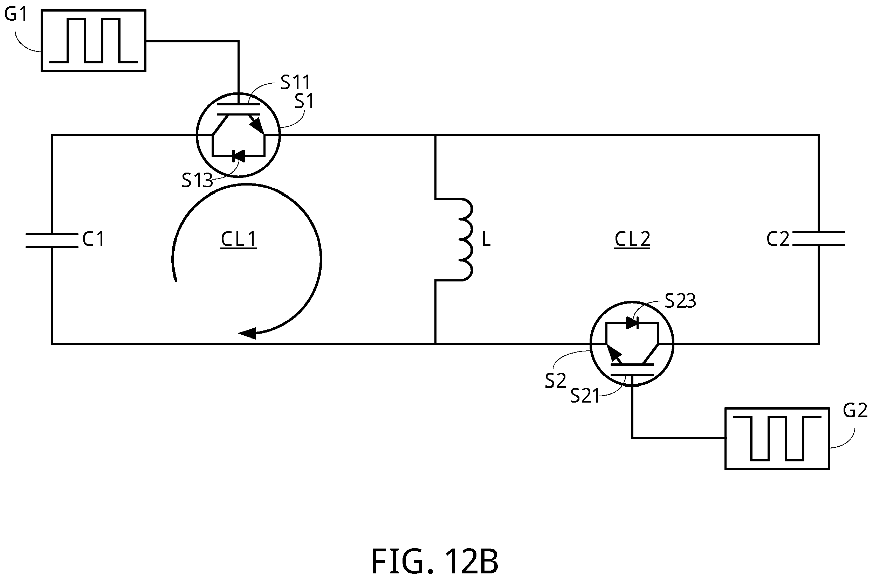

For example, the switch with freewheeling diode may be constituted in the following way: (1) an IGBT connected in parallel with a reverse diode; (2) a single metal oxide semiconductor field effect transistor; and (3) a metal oxide semiconductor field effect transistor first connected in series with a forward diode and then connected in parallel with a reverse diode, but not limited thereto. Practically, the switches may include various components so that the switches are equivalent to an electrical network formed by a controllable switch connected in series with a reverse diode when they are performing the operations of **FIG. 11A** to **FIG. 11B** and **FIG. 12B** to **FIG. 12E**.

The controllable switches **S11** and **S21** may be insulated gate bipolar transistors (IGBTs), metal oxide semiconductor field effect transistors (MOSFETs), gate turn-off thyristors (GTOs), bipolar junction transistors (BJTs), power MOSFETs, MOS-controlled thyristors (MCTs), integrated gate-commutated thyristors (IGCTs), injection enhanced gate transistors (IEGTs) or the like, but not limited thereto.

In other embodiments, the portable rTMS apparatus **1** of the present invention may further comprise a controller to control the first pulse generator **G1** and the second pulse generator **G2**. The inductor **L** is used as a stimulator. The first pulse signal and the second pulse signal enable the first driver circuit loop **CL1** and the second driver circuit loop **CL2** to conduct alternately. The first driver circuit loop **CL1** and the second driver circuit loop **CL2** have magnetic flux driving directions opposite to each other in respect to the inductor **L**. The portable rTMS apparatus **1** of the present invention can generate asymmetrical waveforms by configuration of two energy storage capacitors (i.e., the first energy storage capacitor **C1** and the second energy storage capacitor **C2**) and two freewheeling diode switches (i.e., the first switch with freewheeling diode **S1** and the second switch with freewheeling diode **S2**), thereby achieving a better stimulating effect.

As described previously, the core **COE** of the inductor **L** of the present invention may be a high saturation flux density material to reduce the magnetic resistance (by about 75%) and reduce the current amount (by about 75%) required for generating the magnetic field of the same magnitude. In this case, because the reduction of the current amount may lead to the reduction of the energy stored in the inductor **L**, the present invention may use capacitors having smaller capacitance as the first energy storage capacitor **C1** and the second energy storage capacitor **C2**. The energy storage capacitors are capacitors having an energy storage value greater than 2.5 J, and they are often called bulk capacitors on the scale of circuit in household power suppliers. Practically, the energy required by the inductor is about 25 J. The bulk capacitor is responsible for providing most of the energy supply during the operations as shown in **FIG. 11** to **FIG. 12**, and the capacitance thereof is at least ten times greater than other non-energy storage capacitors on the circuit, e.g., the capacitor of a snubber. The capacitor may be formed by more than one physical capacitor connected in parallel or in series so that the capacitor is still equivalent to a bulk capacitor during the operation of the circuit. For example, a power supplier often uses many small capacitors connected in parallel in order to reduce the impedance of the conductor. The energy storage capacitors mentioned in the subsequent embodiments are all defined in the same way. In practical circuit design, a ratio of a first initial voltage of the first energy storage capacitor **C1** to a second initial voltage of the second energy storage capacitor **C2** may be substantially set to be about 4, and a ratio of a first working period of the first pulse signal to a second working period of the second pulse signal may also be substantially set to be about 4. However, as shall be appreciated by those of ordinary skill in the art, the aforesaid ratios may also be set to range from 1 to 10.

For example, referring to **FIG. 12A** to **FIG. 12E**, it is assumed that the inductance of the inductor **L** is 200 µH, the capacitance of the first energy storage capacitor **C1** is 2.5 µF, the first initial voltage is 3600 V, the turn-on time of the first pulse generator **G1** is 50 µs, and the capacitance of the second energy storage capacitor **C2** is 40 µF, the second initial voltage is 700 V, the turn-on time of the second pulse generator **G2** is 200 µs, and the delay time is 0.001 s. The first initial voltage represents that the first energy storage capacitor **C1** is at the saturated status, and the second energy storage capacitor **C2** is at the half-saturated status. When the first pulse signal generated by the first pulse generator **G1** controls the controllable switch **S11** within the first switch with freewheeling diode **S1** to be turned on within a time interval **T1**, the first energy storage capacitor **C1** is equivalent to a voltage source and starts to discharge so that the first driver circuit loop **CL1** is turned on, and the direction of the current loop after turned on is clockwise, and the current value of the inductor L increases gradually within the time interval **T1**, as shown in **FIG. 12A** and **FIG.** 12B.

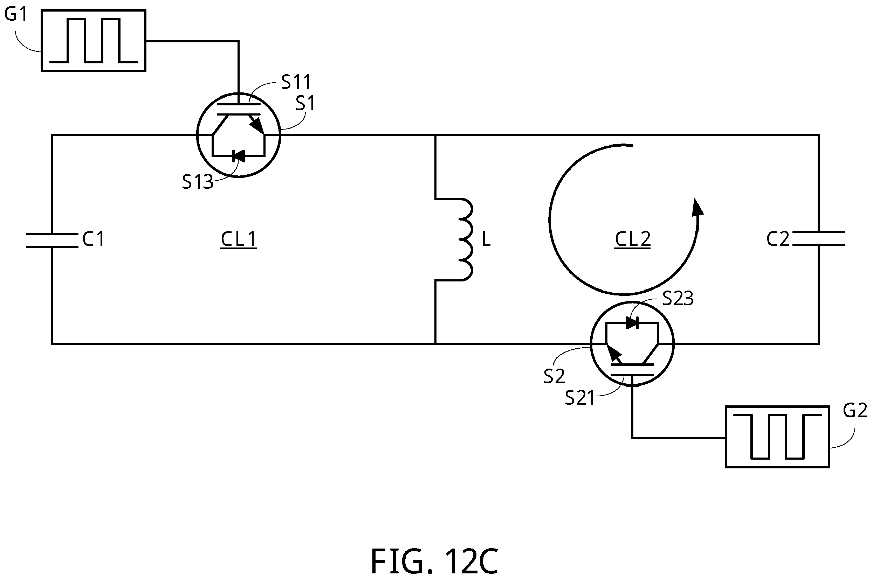

When the time interval **T1** ends, the first pulse signal generated by the first pulse generator **G1** controls the controllable switch **S11** within the first switch with freewheeling diode **S1** to be turned off, and the first driver circuit loop **CL1** is turned off instantly. In response to this, the inductor **L** serves as a current source in the circuit within a time interval **T2** and starts to discharge and the current value thereof decreases gradually within the time interval **T2**. Within the time interval **T2**, the discharge voltage of the inductor **L** enables a diode **S23** to be turned on, so the second driver circuit loop **CL2** is turned on in response to the diode **S23** within the second switch with freewheeling diode **S2**, and the direction of the current loop that is turned on of the second driver circuit loop **CL2** is counterclockwise, as shown in **FIG. 12C**. Moreover, if the second energy storage capacitor **C2** is not at the voltage saturated status within the time interval **T2**, then the second energy storage capacitor **C2** will be charged during the discharging of the inductor **L**.

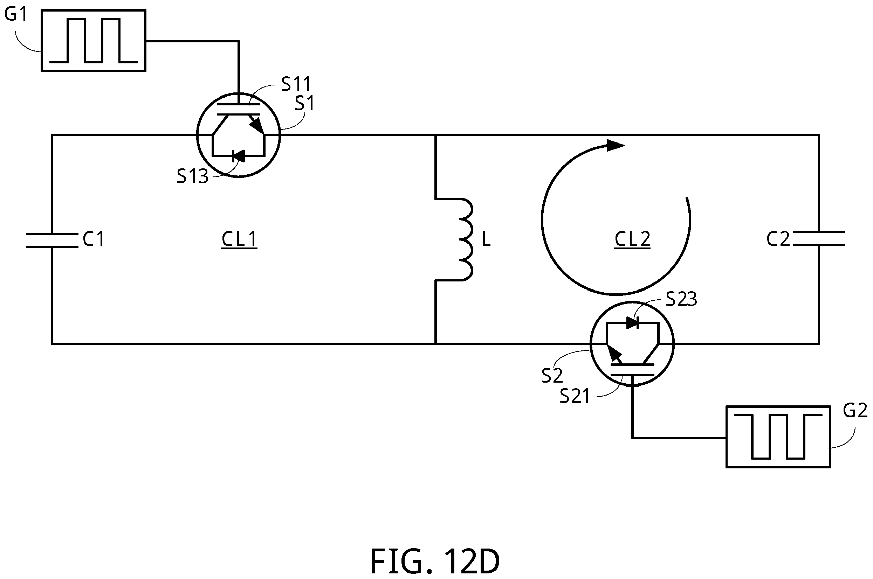

After the time interval **T2** ends (i.e., the inductor **L** is completely discharged), the second pulse signal generated by the second pulse generator **G2** controls the controllable switch **S21** within the second switch with freewheeling diode **S2** to be turned on within a time interval **T3**, the second energy storage capacitor **C2** is equivalent to a voltage source and starts to discharge so that the second driver circuit loop **CL2** is turned on, the direction of the current loop after turned on is clockwise, and the current value of the inductor **L** increases gradually within the time interval **T3**, as shown in **FIG. 12A** and **FIG. 12D**. It shall be appreciated that, the "increase" and "decrease" of the current value herein refer to the "increase" and "decrease" of the absolute values of the current values of the inductor **L**, and do not represent the flowing direction of the current. Therefore, as shall be appreciated by those of ordinary skill in the art, the positive and negative values of the current in **FIG. 12A** represent directions of the current.

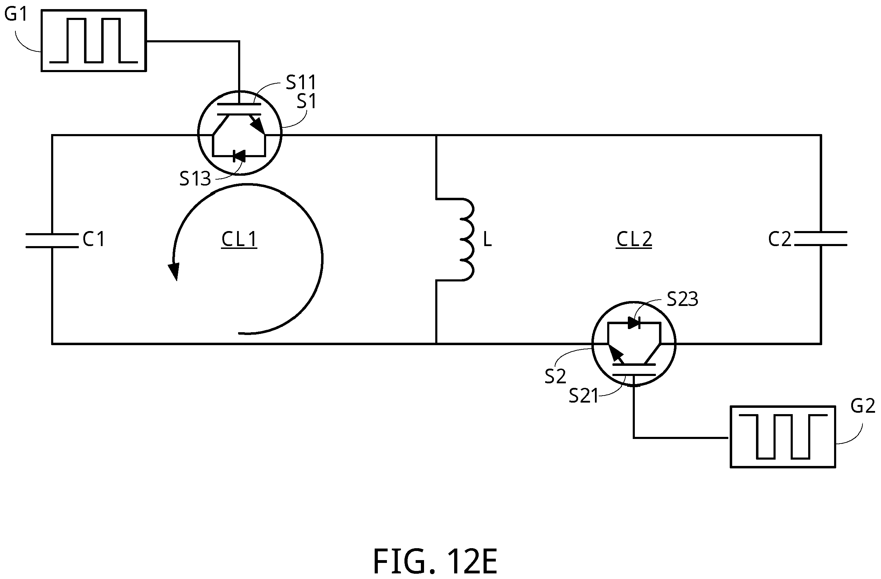

When the time interval **T3** ends, the second pulse signal generated by the second pulse generator **G2** controls the controllable switch **S21** within the second switch with freewheeling diode **S2** to be turned off, and the second driver circuit loop **CL2** is turned off instantly, similar to the time interval **T2**. In response to this, the inductor **L** serves as a current source in the driver circuit within a time interval **T4** and starts to discharge and the current value thereof decreases gradually within the time interval **T4**. Within the time interval **T4**, the discharge voltage of the inductor **L** enables the diode **S13** to be turned on, so the first driver circuit loop **CL1** is turned on in response to the diode **S13** within the first switch with freewheeling diode **S1,** and the direction of the current loop that is turned on of the first driver circuit loop **CL1** is counterclockwise, as shown in **FIG. 12E**. If the first energy storage capacitor **C1** is not at the voltage saturated status when the inductor **L** is discharging, then the first energy storage capacitor **C1** will be charged during the discharging of the inductor **L**. **T1** to **T4** is one complete charge-and-discharge period, and one period consumes time about 250 µs, and there may be 5 to 20 periods within one second, each of which is 250 µs and distributed uniformly between one second. For a bidirectional waveform of 250 µs, an electric field of 84 V/M is required at a depth of 25 mm, so a maximum current peak can be determined according to the value.

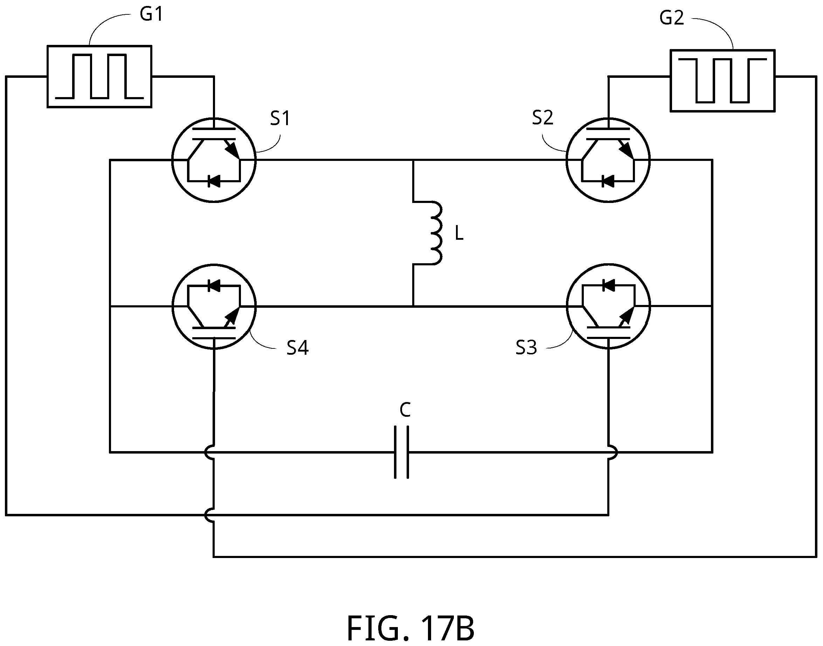

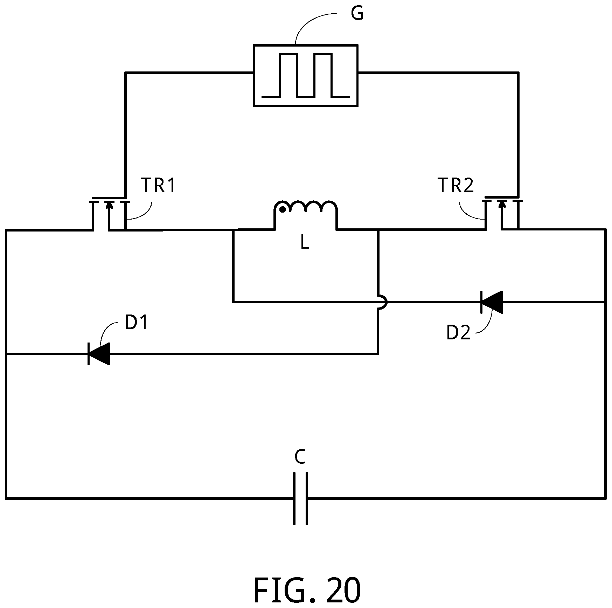

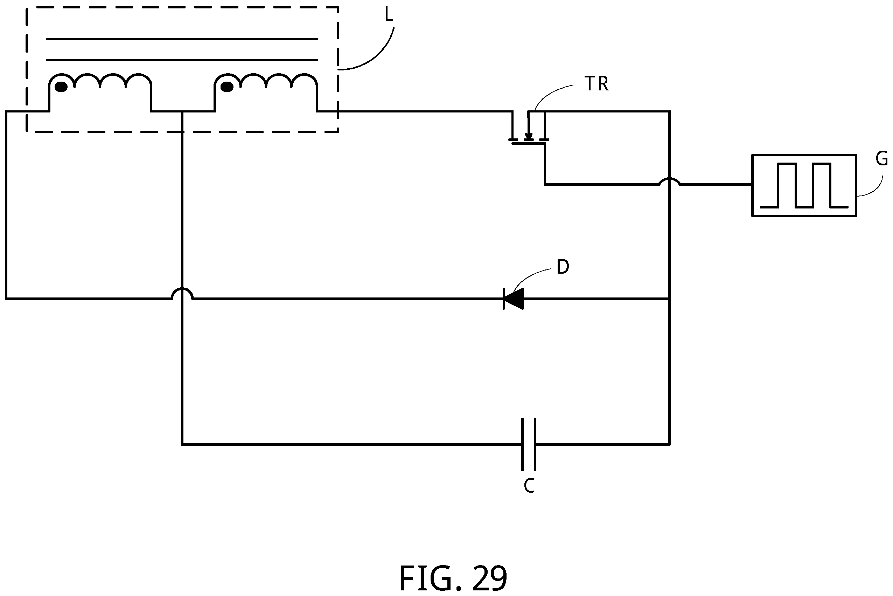

It shall be appreciated that, the aforesaid embodiments are all described as an extension of the circuit of **FIG. 12A****.** However, the operation of the circuit shown in **FIG. 12B** shall be appreciated by those of ordinary skill in the art based on the above description. For example, the operations in **FIG. 23** are similar to **T1** to **T4**. The operations of **FIG. 20** and **FIG. 29** are similar to **T1** to **T2**. If the operations of **FIG. 17B** and **FIG. 26** control the pulse generators **G1** and **G2**, then the two pulse signals will make it being similar to **T1** to **T4**; and if the operations of **FIG. 17B** and **FIG. 26** only control the pulse generator **G1**, then a single pulse signal will make it being similar to **T1** to **T2**. In **FIG. 17B** and **FIG. 20****,** because there are only a single bulk capacitor and a single stimulator, only a symmetrical waveform where **T1=T2**, or **T3=T4** can be generated, which is different from the case of **FIG. 12A**. The above circuit operation may be regarded as a discontinuous conduction mode (DCM) in a power electronic converter.

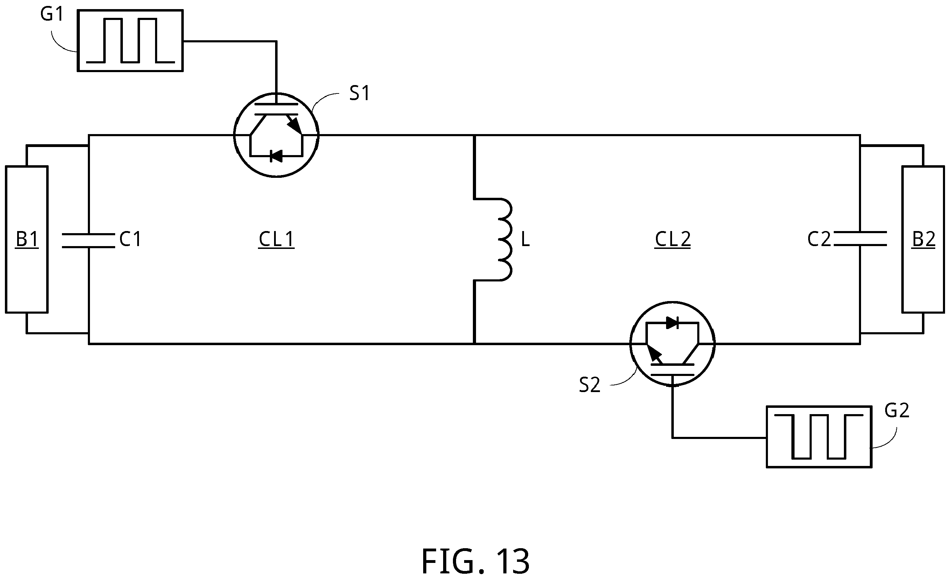

In other embodiments, the first energy storage capacitor **C1** may be coupled to a first voltage booster **B1** to charge the first energy storage capacitor **C1** when the first driver circuit loop **CL1** is at the conducting or non-conducting period (e.g., after the time interval **T4**, before the next time interval **T1**), and the second energy storage capacitor **C2** may be coupled to a second voltage booster **B2** to charge the second energy storage capacitor **C2** when the second driver circuit loop **CL2** is at the conducting or non-conducting period (e.g., after the time interval **T2**, before the time interval **T3**, i.e., at the time interval **T5**), as shown in **FIG. 13**. Additionally, in other embodiments, the first energy storage capacitor **C1** may be set to be coupled to the first voltage booster **B1**, and the second energy storage capacitor **C2** is not coupled to any voltage booster; or the first energy storage capacitor **C1** is not coupled to any voltage booster, and the second energy storage capacitor **C2** is coupled to the second voltage booster **B2**. The first voltage booster **B1** and the second voltage booster **B2** may be connected to a power supply module (not shown), and the power supply module may be connected to an internal battery of the portable rTMS apparatus, or connected to an external power supply (e.g., a wall outlet or a mobile power supply) via an interface.

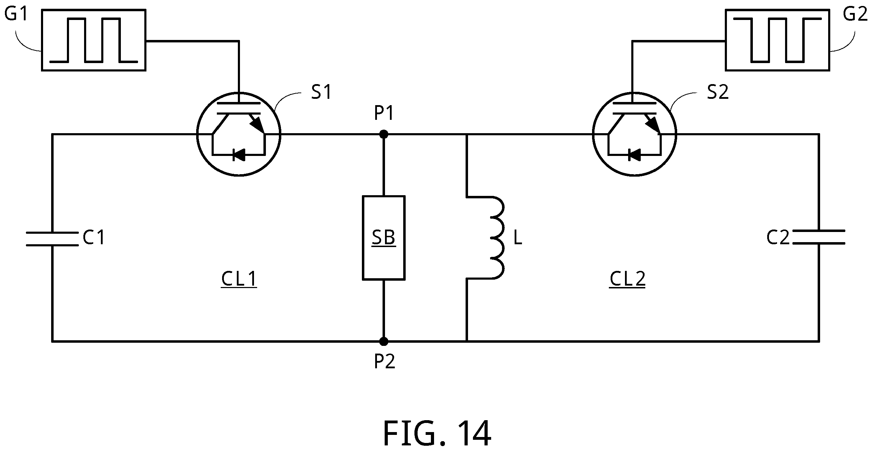

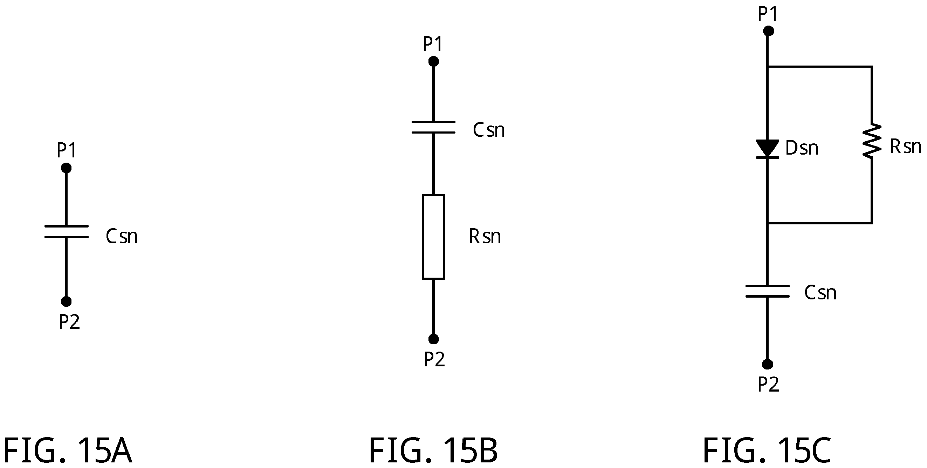

Moreover, in other embodiments, the driver circuit **DRC** further comprises at least one snubber (e.g., a passive snubber or a semi-active snubber). For example, as shown in **FIG. 14**, the driver circuit **DRC** comprises a passive snubber **SB** bridging two sides of the inductor **L**. In **FIG. 15A**, the passive snubber **SB** is constituted by a single capacitor **Csn**. In **FIG. 15B**, the passive snubber **SB** is constituted by a capacitor **Csn** and a resistor **Rsn** that are connected in series. In **FIG. 15C**, the passive snubber **SB** is constituted by connecting a diode **Dsn** and a resistor **Rsn** in parallel which are then connected with the capacitor **Csn** in series. The passive snubber **SB** is defined as any of the structures of **FIG. 15A** to **FIG. 15C**, and the passive snubbers **SB** mentioned in the subsequent embodiments are all defined in the same way.

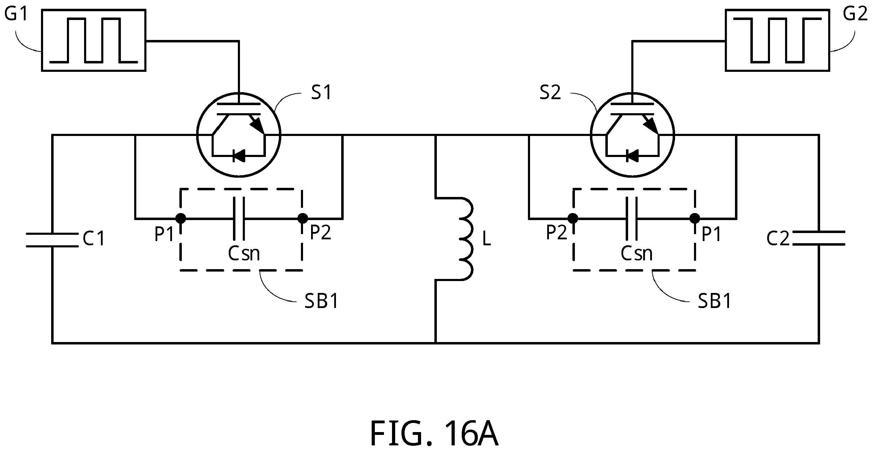

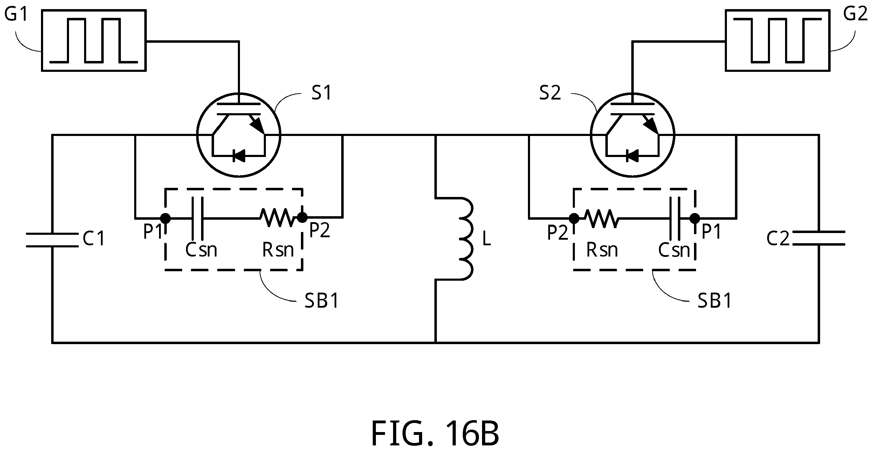

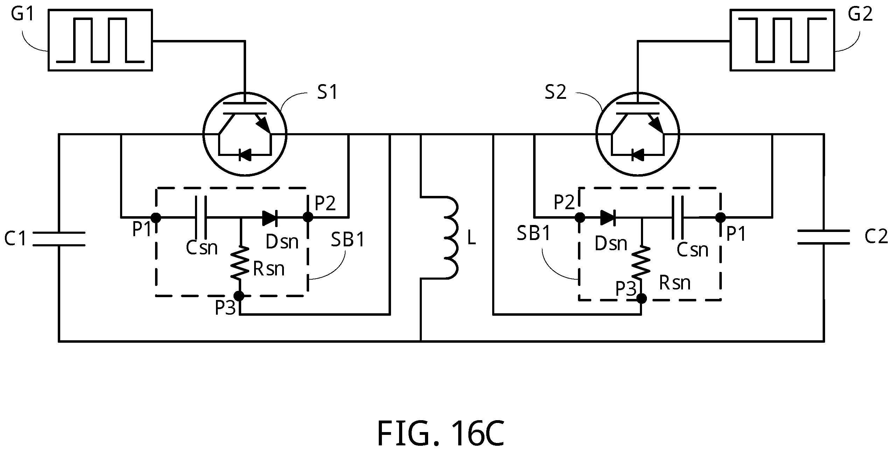

For example, the driver circuit **DRC** may comprise two passive snubbers **SB1** that are respectively connected in parallel with the first switch with freewheeling diode **S1** and the second switch with freewheeling diode **S2**, as shown in **FIG. 16A** to **FIG. 16C**. The passive snubbers **SB** and **SB1** can be used to absorb the surge in the driver circuit **DRC**, thereby preventing the driver circuit **DRC** from being damaged due to the surge.

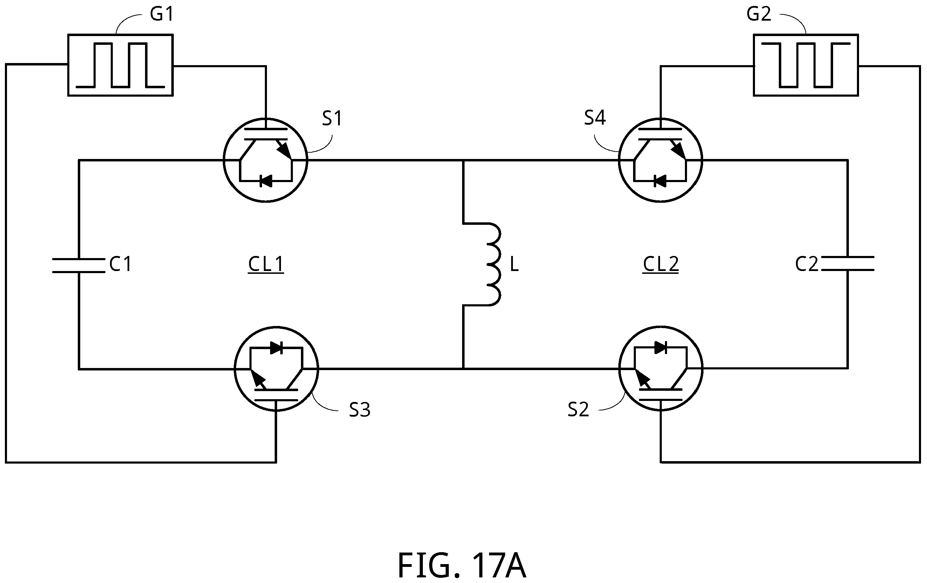

Moreover, in the driver circuit **DRC**, if a first auxiliary switch with freewheeling diode **S3** is added, or a second auxiliary switch with freewheeling diode **S4** is added, or both the first auxiliary switch with freewheeling diode **S3** and the second auxiliary switch with freewheeling diode **S4** are added, then as shown in **FIG. 17A**, the circuit operation will not be influenced according to the aforesaid operational principle. The difference lies in that, if both the first auxiliary switch with freewheeling diode **S3** and the second auxiliary switch with freewheeling diode **S4** are added, then the common ground type of the two energy storage capacitors can be changed. In other words, the first energy storage capacitor and the second energy storage capacitor originally can only have the positive voltage connected to the negative voltage, and after adopting the four switches, the positive ends may be connected together or the negative ends may be connected together.

A third embodiment of the present invention is as shown in **FIG. 17B**, which is an extension of **FIG. 17A** in the second embodiment. In **FIG. 17B**, the first driver circuit loop **CL1** is formed by the inductor **L**, the energy storage capacitor **C**, the first switch with freewheeling diode **S1** and the first auxiliary switch with freewheeling diode **S3** that are connected in series, and the second driver circuit loop **CL2** is formed by the inductor **L**, the energy storage capacitor **C**, the second switch with freewheeling diode **S2** and the second auxiliary switch with freewheeling diode **S4** that are connected in series. The aforesaid connection mode is defined as "a connection mode of a full-bridge primary side".

The first driver circuit loop **CL1** comprises the first pulse generator **G1** that is coupled to the first switch with freewheeling diode **S1** and the first auxiliary switch with freewheeling diode **S3** and generating a first pulse signal to control the first switch with freewheeling diode **S1** and the first auxiliary switch with freewheeling diode **S3**. The second driver circuit loop **CL2** comprises the second pulse generator **G2** coupled to the second switch with freewheeling diode **S2** and the second auxiliary switch with freewheeling diode **S4** and generating a second pulse signal to control the second switch with freewheeling diode **S2** and the second auxiliary switch with freewheeling diode **S4**. The "auxiliary switch with freewheeling diode" is defined as: another switch with freewheeling diode at a position opposite to the switch with freewheeling diode in respect to the inductor in a driver loop and the energy storage capacitor in the driver loop. The control signal received by the auxiliary switch with freewheeling diode will be the same as the control signal received by the switch with freewheeling diode.

Similarly, the portable rTMS apparatus **1** may further comprise a controller to control the first pulse generator **G1** and the second pulse generator **G2**. The first pulse signal and the second pulse signal enable the first driver circuit loop and the second driver circuit loop to conduct alternately. The first driver circuit loop **CL1** and the second driver circuit loop **CL2** have magnetic flux driving directions opposite to each other in respect to the inductor **L**.

Similarly, the portable rTMS apparatus **1** may also further comprise a voltage booster (not shown) that is coupled to the capacitor **C** to charge the capacitor **C** when the first driver circuit loop **CL1** and the second driver circuit loop **CL2** are all at a non-conducting period. Moreover, the voltage booster may be connected to a power supply module (not shown), and the power supply module may be connected to an internal battery of the portable rTMS apparatus, or connected to an external power supply (e.g., a wall outlet or a mobile power supply) via an interface.

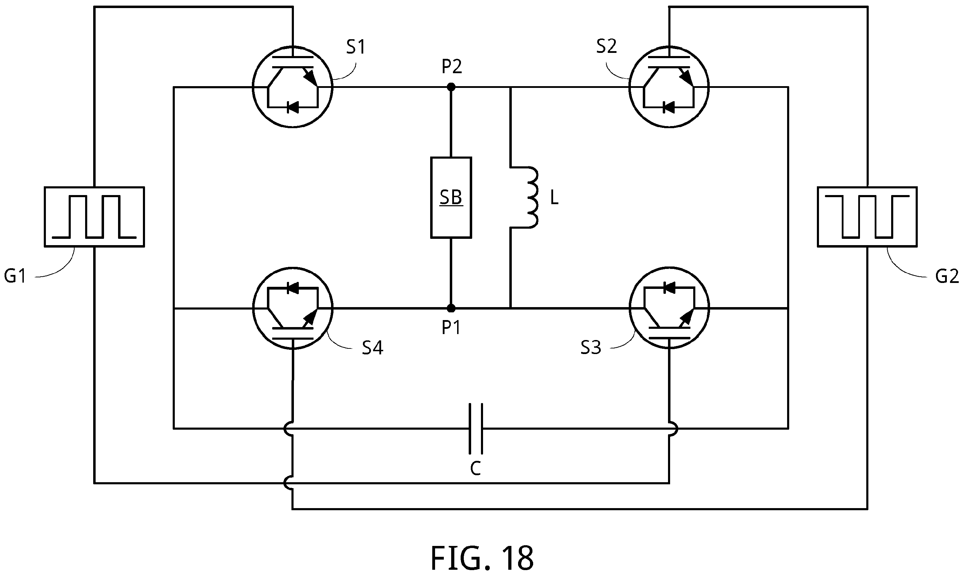

In other embodiments, the driver circuit **DRC** may further comprise at least one passive snubber. For example, referring to **FIG. 18**, the driver circuit **DRC** comprises the passive snubber **SB** bridging two sides of the inductor **L**.

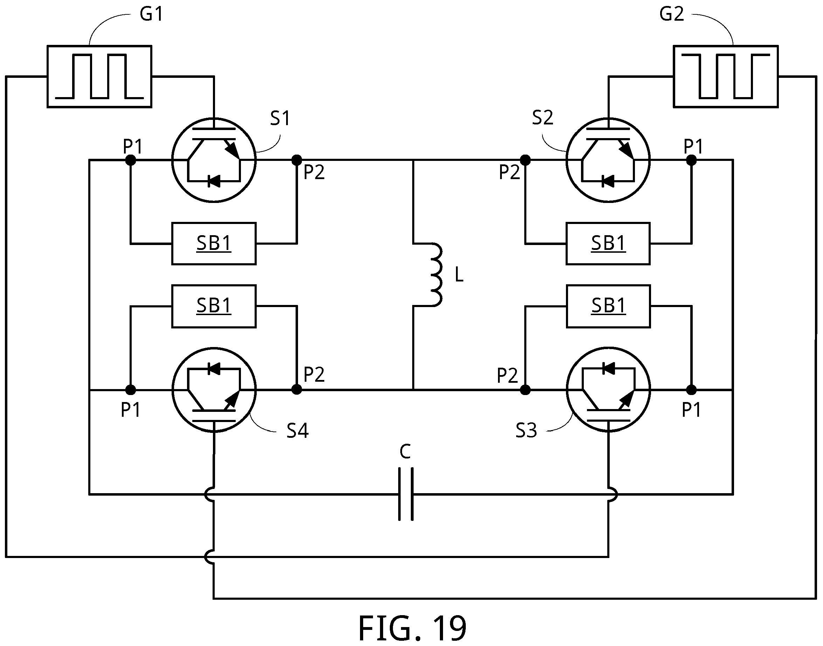

As another example, referring to **FIG. 19**, the driver circuit **DRC** comprises four passive snubbers **SB1**, and the passive snubbers **SB1** are respectively connected with the first switch with freewheeling diode **S1,** the second switch with freewheeling diode **S2**, the first auxiliary switch with freewheeling diode **S3** and the second auxiliary switch with freewheeling diode **S4** in parallel. The snubbers **SB1** can be used to absorb the surge in the driver circuit **DRC**, thereby preventing the driver circuit **DRC** from being damaged due to the surge.

A fourth embodiment of the present invention is as shown in **FIG. 20**, which is an extension of the third embodiment. **FIG. 20** is a schematic view depicting a circuit of the portable rTMS apparatuses **1** within the present invention. The driver circuit **DRC** comprises an energy storage capacitor **C**, a switch **TR1**, an auxiliary switch **TR2**, a first freewheeling diode **D1**, a second freewheeling diode **D2**, and a pulse generator **G**. The aforesaid connection mode is defined as "a connection mode of a primary side of two switch converters".

The pulse generator **G** is coupled to the switch **TR1** and the auxiliary switch **TR2** and generating a pulse signal to simultaneously control the switch **TR1** and the auxiliary switch **TR2**. The inductor **L**, the energy storage capacitor **C**, the switch **TR1** and the auxiliary switch **TR2** are connected in series to form a driver circuit loop, and the inductor **L**, the first freewheeling diode **D1** and the second freewheeling diode **D2** are connected in series to form a re-charging loop. The "switch" is defined as: an electrical network that is equivalent to a controllable switch on scale of circuit in a household power suppliers. The switch may turn on forward current when a control signal is activated, and it may block the forward voltage when the control signal is deactivated. The term of "freewheeling diode" is explained in the following way: when the driver loop is turned off, the diode of the charging loop is immediately turned on, and thus it is called a freewheeling diode. The "auxiliary switch" is defined as: another switch at a position opposite to the switch in respect to the inductor in a driver loop and the energy storage capacitor in the loop. The control signal received by the auxiliary switch will be the same as that of the switch. The "diode" is defined as: a passive component that can block the reverse voltage and turn on the forward current, or an electrical network that is equivalent to the aforesaid component during the operation of the power supplier circuit. The synchronous rectifier is a technology that can replace the diode and reduce the power consumption, and the principle thereof is to appropriately control the active switch (e.g., MOSFET, BJT or the like) to adjust the time periods where the voltage is blocked and the current is turned on. Therefore, it is equivalent to a diode during the operation of the circuit. Therefore, the synchronous rectifier can be regarded as a diode.

Similarly, the portable rTMS apparatus **1** may further comprise a voltage booster (not shown) that is coupled to the capacitor **C** to charge the energy storage capacitor **C** when the driver circuit loop is at a non-conducting period. Moreover, as described in the aforesaid embodiments, the voltage booster may be connected to a power supply module (not shown), and the power supply module (not shown) may be connected to an internal battery of the portable rTMS apparatus, or connected to an external power supply (e.g., a wall outlet or a mobile power supply) via an interface.

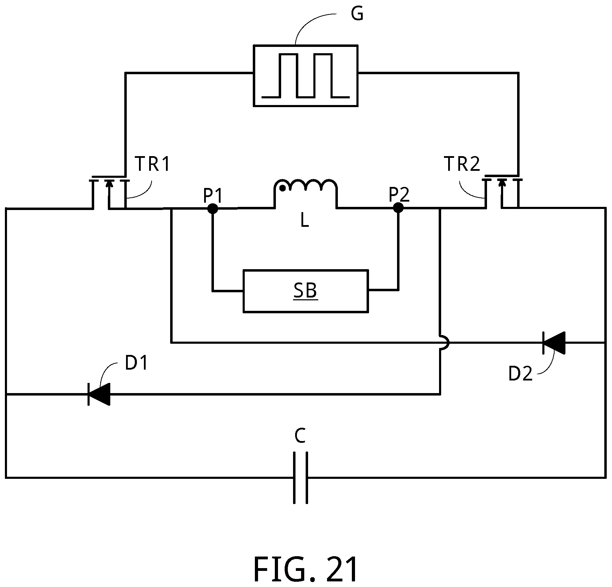

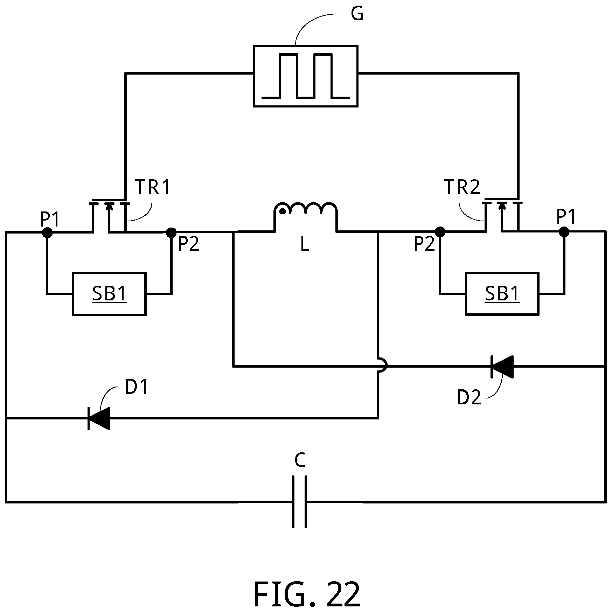

In other embodiments, the driver circuit **DRC** further comprises at least one passive snubber. For example, referring to **FIG. 21**, the driver circuit **DRC** comprises the passive snubber **SB** bridging two sides of the inductor **L**. As another example, in other embodiments, referring to **FIG. 22**, the driver circuit **DRC** comprises two passive snubbers **SB1** that respectively connected in parallel with the switch **TR1** and the auxiliary switch **TR2**.

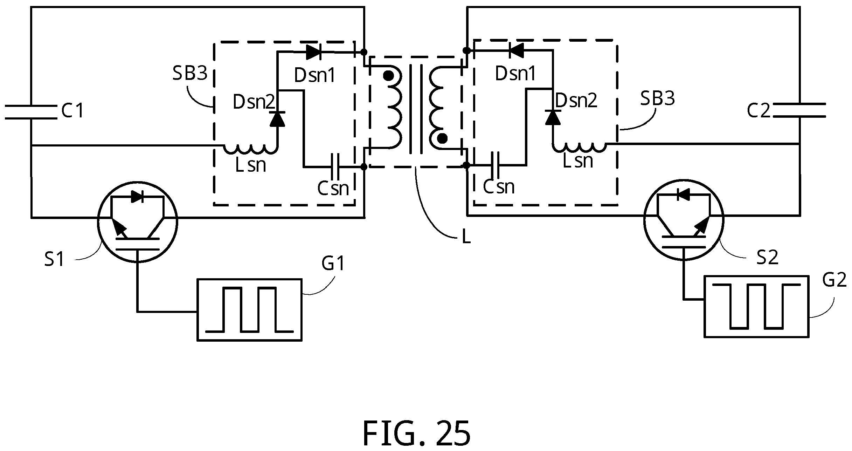

A fifth embodiment of the present invention is as shown in **FIG. 23**, which is an extension of the second embodiment. **FIG. 23** is a schematic view depicting a circuit of the portable rTMS apparatuses **1** within the present invention. The driver circuit **DRC** comprises a first energy storage capacitor **C1**, a second energy storage capacitor **C2**, a first switch with freewheeling diode **S1**, a second switch with freewheeling diode **S2**, a first pulse generator **G1** and a second pulse generator **G2** which are presented in the form similar to a flyback converter. Similarly, the first energy storage capacitor **C1**, the first switch with freewheeling diode **S1** and a primary coil of the inductor **L** are connected in series to form the first driver circuit loop **CL1,** and the second energy storage capacitor **C2**, the second switch with freewheeling diode **S2**, and the secondary coil of the inductor **L** are connected in series to form the second driver circuit loop **CL2**. It shall be appreciated that, the schematic view of the circuit in **FIG. 25** is not intended to limit the sequence of the first energy storage capacitor **C1** and the first switch with freewheeling diode **S1** in the first driver circuit loop **CL1** and the sequence of the second energy storage capacitor **C2** and the second switch with freewheeling diode **S2** in the second driver circuit loop **CL2**. In practical application, the provider or the manufacturer of the portable rTMS apparatus **1** may decide the order in which the capacitors and the switches are connected in series according to different requirements. The aforesaid connection mode is defined as "a connection mode of a bidirectional flyback converter".

The primary coil of the inductor **L** and the secondary coil of the inductor **L** being the two coils of a stimulator. The first pulse signal and the second pulse signal enable the first driver circuit loop **CL1** and the second driver circuit loop **CL2** to be conduct alternately. The first driver circuit loop **CL1** and the second driver circuit loop **CL2** have magnetic flux driving directions opposite to each other in respect to the inductor **L**.

Similarly, the first energy storage capacitor **C1** may be coupled to a voltage booster (not shown) to charge the first energy storage capacitor **C1** when the first driver circuit loop **CL1** is at a non-conducting period, and the second energy storage capacitor **C2** may also be coupled to a voltage booster (not shown) to charge the second energy storage capacitor **C2** when the second driver circuit loop **CL2** is at a non-conducting period. Moreover, in other embodiments, the voltage booster coupled to the first energy storage capacitor **C1** and the voltage booster coupled to the second energy storage capacitor **C2** may be connected to a power supply module (not shown), and the power supply module may be connected to an internal battery of the portable rTMS apparatus, or connected to an external power supply (e.g., a wall outlet or a mobile power supply) via an interface.

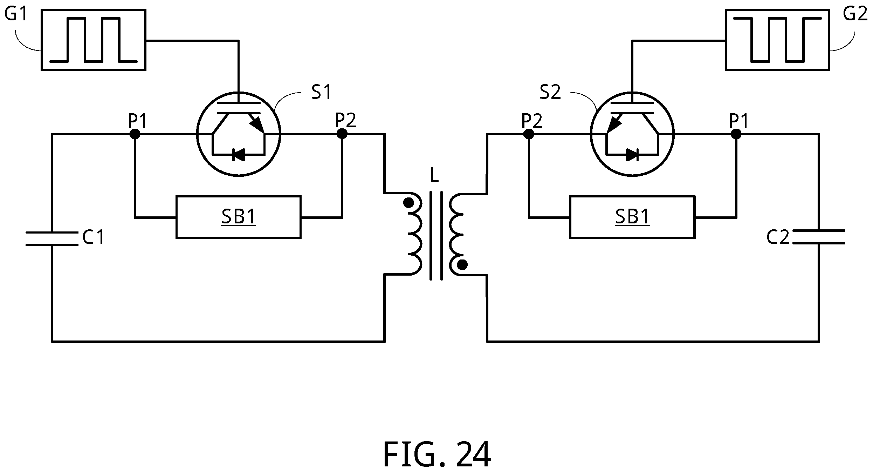

Moreover, in other embodiments, the driver circuit **DRC** further comprises at least one passive snubber connected to the first switch with freewheeling diode **S1** and the second switch with freewheeling diode **S2**. For example, as shown in **FIG. 24**, two passive snubbers **SB1** are respectively connected in parallel with the first switch with freewheeling diode **S1** and the second switch with freewheeling diode **S2**.

Moreover, in other embodiments, the driver circuit **DRC** may comprise an energy-recovering snubber **SB3** as shown in **FIG. 25**. The "energy-recovering snubber" is defined as a snubber constituted by diodes **Dsn1** and **Dsn2**, an inductor **Lsn** and a capacitor **Csn**. In the first driver circuit loop **CL1**, the diodes **Dsn1** and **Dsn2**, the inductor **Lsn** and the energy storage capacitor **C1** are connected in series to form a loop, and the diodes **Dsn1** and **Dsn2** are connected to an endpoint between the inductor **L** and the first switch with freewheeling diode **S1** via a capacitor **Csn** therebetween. The second driver circuit loop **CL2** is connected in the same way. The energy-recovering snubbers **SB3** mentioned in the subsequent embodiments are all defined in the same way. Additionally, in other embodiments, a coupling capacitor may bridge two sides of the inductor **L**, and when the coupling capacitance is very small, the function of the coupling capacitor in the driver circuit **DRC** is equivalent to a snubber that is configured to filter the surge in the circuit. The first energy storage capacitor **C1** and the second energy storage capacitor **C2** may be common grounded in various manners, e.g., connecting positive ends together, connecting positive ends and negative ends together or the like, all of which belong to reasonable circuit types. It shall be appreciated that, the inductor **Lsn** refers to an inductor that is specially added other than the stimulator, and the inductor **L** in other figures all refers to the stimulator.

A sixth embodiment of the present invention is as shown in **FIG. 26**. **FIG. 26** is a schematic view depicting a circuit of the portable rTMS apparatuses **1** within the present invention. The driver circuit **DRC** comprises an energy storage capacitor **C**, a first switch with freewheeling diode **S1**, a second switch with freewheeling diode **S2**, a first pulse generator **G1** and a second pulse generator **G2** which are presented in the form similar to a push-pull converter. The aforesaid connection mode is defined as "a connection mode of a primary side of a push-pull converter". **FIG. 26** is represented by a center-tapped inductor, which can also be equivalent to two coupling inductors common grounded, and then the two switches with freewheeling diode may be modified to be located at opposite sides of the capacitor.

In this embodiment, the first driver circuit loop **CL1** is formed by connecting the primary coil of the inductor **L**, the energy storage capacitor **C** and the first switch with freewheeling diode **S1** in series, and the second driver circuit loop **CL2** is formed by connecting the secondary coil of the inductor **L**, the energy storage capacitor **C** and the second switch with freewheeling diode **S2** in series. Similarly, the first pulse signal generated by the first pulse generator **G1** and the second pulse signal generated by the second pulse generator **G2** enable the first driver circuit loop **CL1** and the second driver circuit loop **CL2** to conduct alternately, and the first driver circuit loop **CL1** and the second driver circuit loop **CL2** have magnetic flux driving directions opposite to each other in respect to the inductor. Similarly, in other embodiments, the portable rTMS apparatus **1** of the present invention may further comprise a controller for controlling the first pulse generator **G1** and the second pulse generator **G2**.

Similarly, the portable rTMS apparatus may further comprise a voltage booster (not shown) that is coupled to the capacitor **C** to charge the energy storage capacitor **C** when the first driver circuit loop and the second driver circuit loop are all at a non-conducting period. The voltage booster may be connected to a power supply module (not shown), and the power supply module may be connected to an internal battery of the portable rTMS apparatus, or connected to an external power supply (e.g., a wall outlet or a mobile power supply) via an interface.

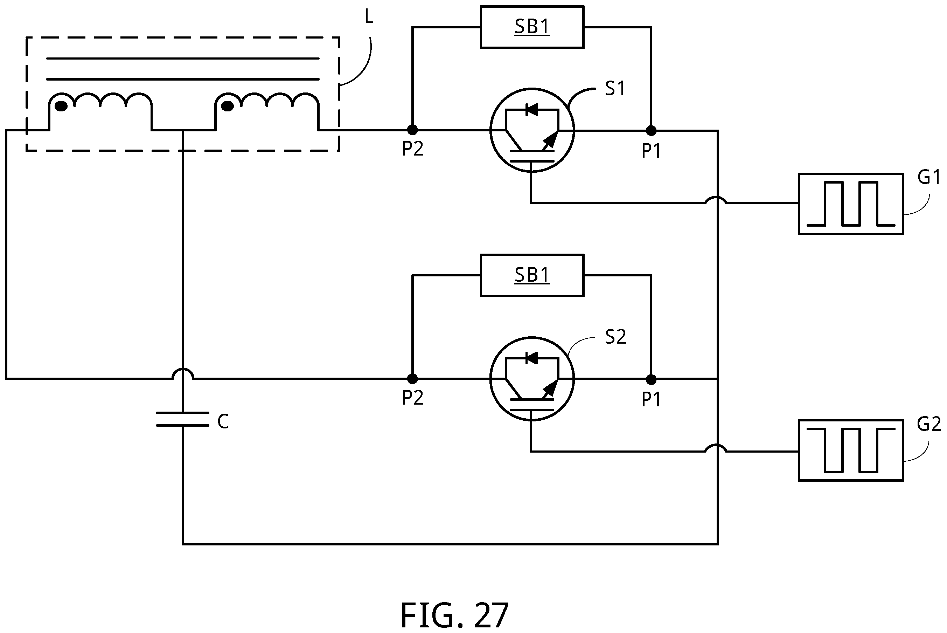

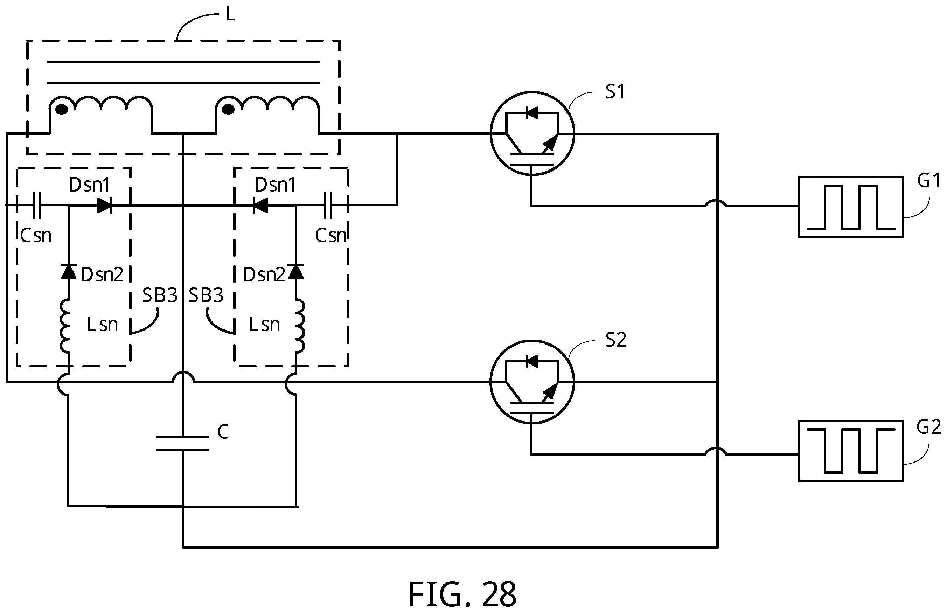

In other embodiments, the driver circuit **DRC** further comprises at least one passive snubber connected to the first switch with freewheeling diode **S1** and the second switch with freewheeling diode **S2**. For example, as shown in **FIG. 27**, two passive snubbers SB1 are respectively connected with the first freewheeling diode switch **S1** and the second switch with freewheeling diode **S2** in parallel. Moreover, in other embodiments, the driver circuit **DRC** of this embodiment may also adopt the energy-recovering snubber **SB3** of the aforesaid embodiments, as shown in **FIG. 28**.

A seventh embodiment of the present invention is as shown in **FIG. 29**. **FIG. 29** is a schematic view depicting a circuit of the portable rTMS apparatuses **1** within the present invention. The driver circuit **DRC** comprises an energy storage capacitor **C**, a switch **TR**, a freewheeling diode **D**, and a pulse generator **G** which are presented in the form similar to a push-pull converter. The pulse generator **G** is coupled to the switch **TR** and generating a pulse signal to control the switch **TR**. When the pulse generator **G** controls to turn on the switch **TR**, the inductor **L**, the energy storage capacitor **C** and the switch **TR** connected in series form a driver circuit loop. When the pulse generator **G** controls to turn off the switch **TR**, the inductor **L**, the energy storage capacitor **C** and the freewheeling diode **D** connected in series form a re-charging loop. The aforesaid connection mode is defined as "a connection mode of the primary side of a simplified push-pull converter". **FIG. 29** is represented by a center-tapped inductor, which may also be equivalent to two coupling inductors common grounded, and then the freewheeling diode switch and the diode may be modified to be located at opposite sides of the capacitor.

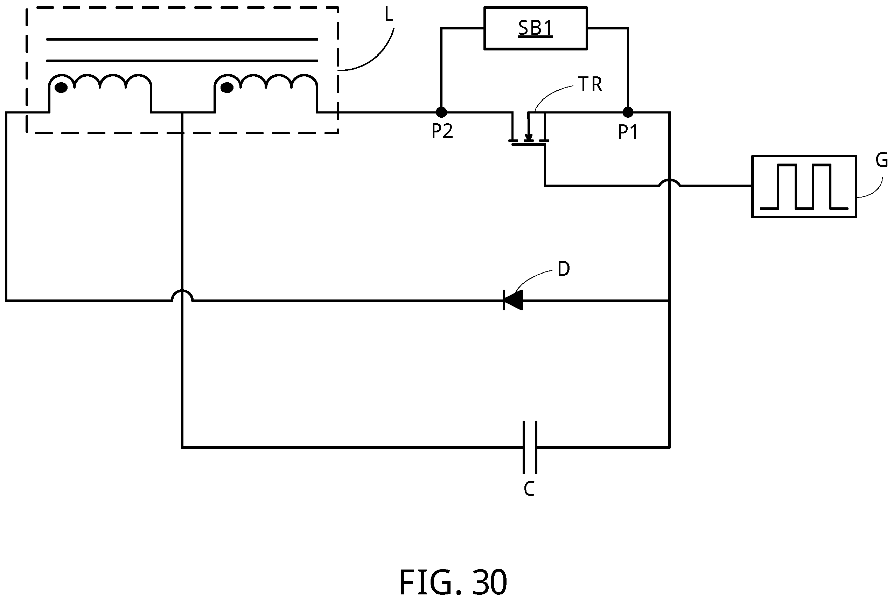

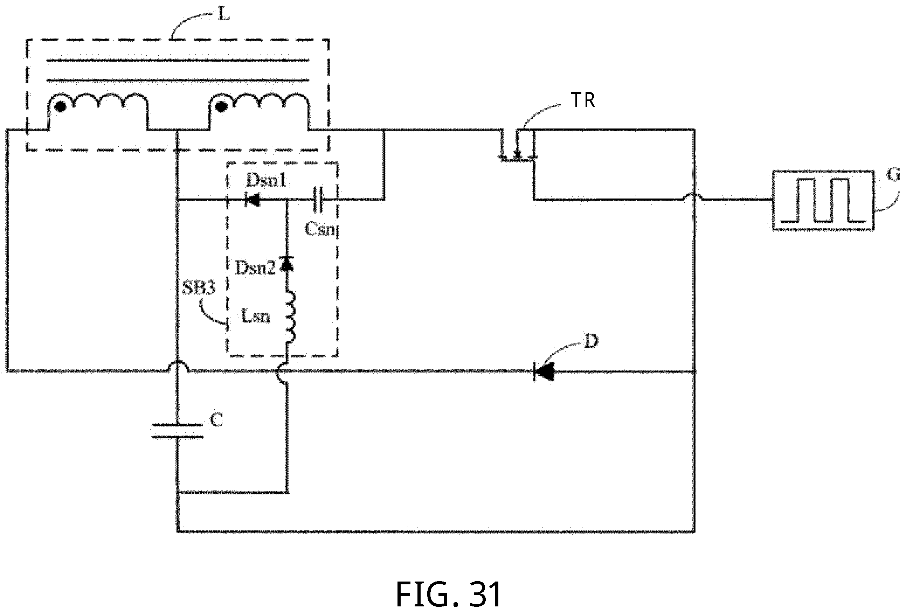

Similarly, the portable rTMS apparatus may further comprise a voltage booster (not shown) that is coupled to the energy storage capacitor C to charge the energy storage capacitor **C** when the driver circuit loop **DRC** is at a non-conducting period. In other embodiments, the driver circuit **DRC** further comprises the passive snubber **SB1** connected to the switch **TR**, as shown in **FIG. 30**. Moreover, in other embodiments, the driver circuit **DRC** of this embodiment may also adopt the energy-recovering snubber **SB3** of the aforesaid embodiments, as shown in **FIG. 31**.

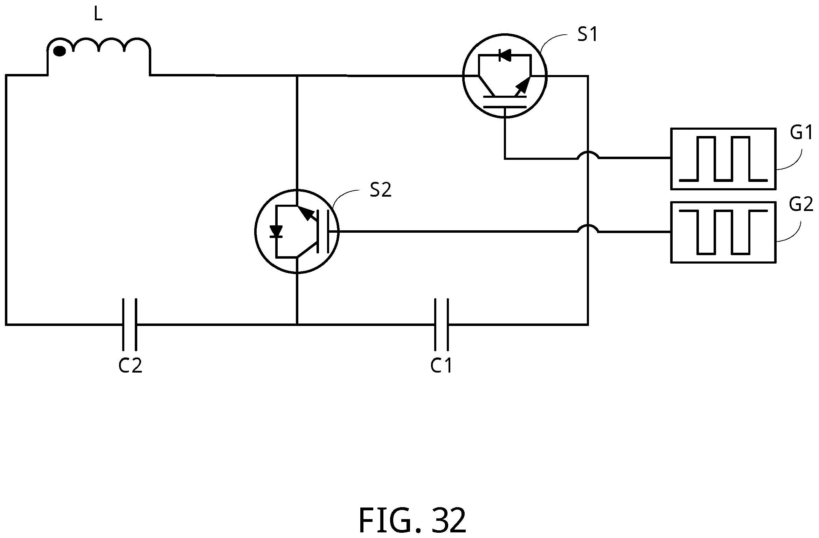

An eighth embodiment of the present invention is as shown in **FIG. 32**. **FIG. 32** is a schematic view depicting a circuit of the portable rTMS apparatuses **1** within the present invention. The driver circuit **DRC** comprises a first energy storage capacitor **C1**, a second energy storage capacitor **C2**, a first switch with freewheeling diode **S1**, a second switch with freewheeling diode **S2**, a first pulse generator **G1** and a second pulse generator **G2**. In this embodiment, the first driver circuit loop **CL1** is formed by connecting the inductor **L**, the first energy storage capacitor **C1**, the second energy storage capacitor **C2** and the first switch with freewheeling diode **S1** in series, and the second driver circuit loop **CL2** is formed by connecting the inductor **L**, the second energy storage capacitor **C2** and the second switch with freewheeling diode **S2** in series. The aforesaid connection mode is defined as "a connection mode of a bidirectional battery charger".

The first pulse generator **G1** is coupled to the first switch with freewheeling diode **S1** and generating a first pulse signal to control the first switch with freewheeling diode **1**. The second pulse generator **G2** is coupled to the second switch with freewheeling diode **S2** and generating a second pulse signal to control the second switch with freewheeling diode **S2**. Similarly, in other embodiments, the portable rTMS apparatus **1** of the present invention may further comprise a controller for controlling the first pulse generator **G1** and the second pulse generator **G2**. The first pulse signal and the second pulse signal enable the first driver circuit loop **CL1** and the second driver circuit loop **CL2** to conduct alternately. The first driver circuit loop **CL1** and the second driver circuit loop **CL2** have magnetic flux driving directions opposite to each other. The operation mode of this embodiment is the same as the bidirectional battery charger of the well-known technology, and the principle thereof is different from the second to the seventh embodiments.

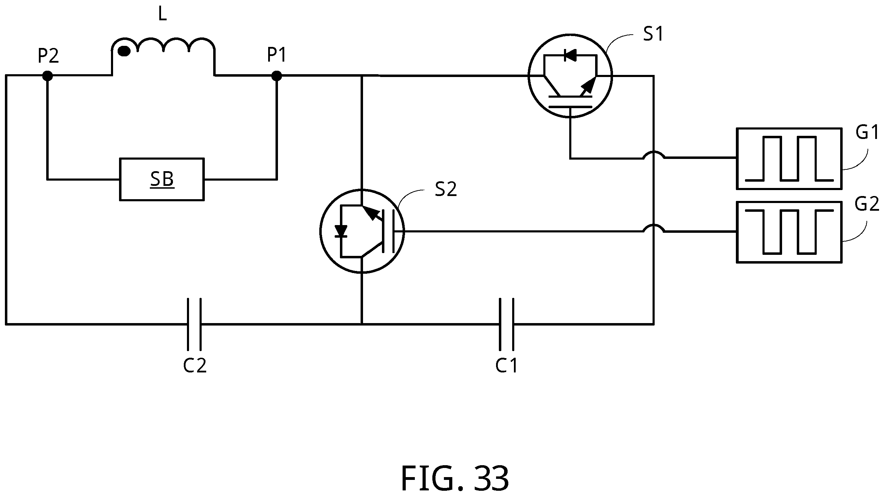

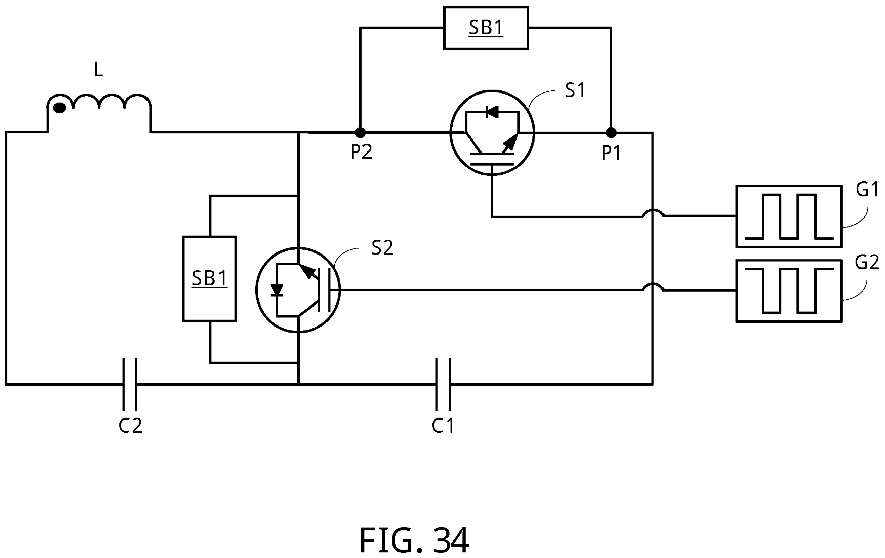

Like the aforesaid embodiments, in the driver circuit **DRC** of this embodiment, the first energy storage capacitor **C1** may be coupled to a voltage booster (not shown) to charge the first capacitor **C1** when the first driver circuit loop is at a non-conducting period, and the second energy storage capacitor **C2** may be coupled to another voltage booster (not shown) to charge the second capacitor **C2** when the second driver circuit loop is at a non-conducting period. Moreover, in other embodiments, the voltage booster may be connected to a power supply module (not shown), and the power supply module may be connected to an internal battery of the portable rTMS apparatus, or connected to an external power supply (e.g., a wall outlet or a mobile power supply) via an interface. Additionally, in other embodiments, the driver circuit **DRC** further comprises at least one passive snubber. For example, referring to **FIG. 33**, the driver circuit **DRC** comprises the passive snubber **SB** bridging two sides of the inductor **L**. As another example, in other embodiments, referring to **FIG. 34**, the driver circuit **DRC** comprises two passive snubbers **SB1** that are respectively connected in parallel with the first switch with freewheeling diode **S1** and the second switch with freewheeling diode **S2**.

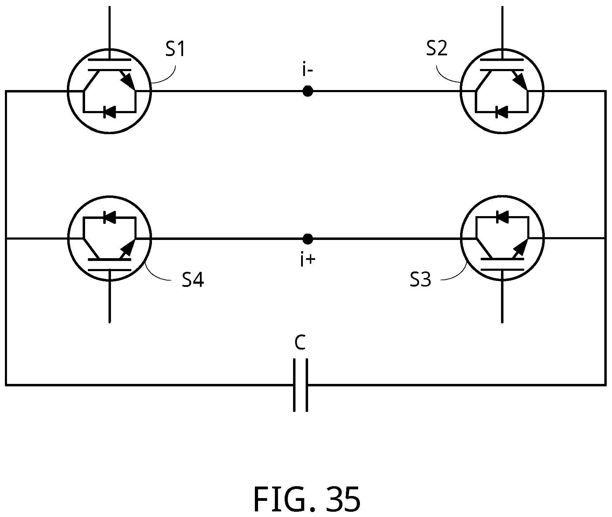

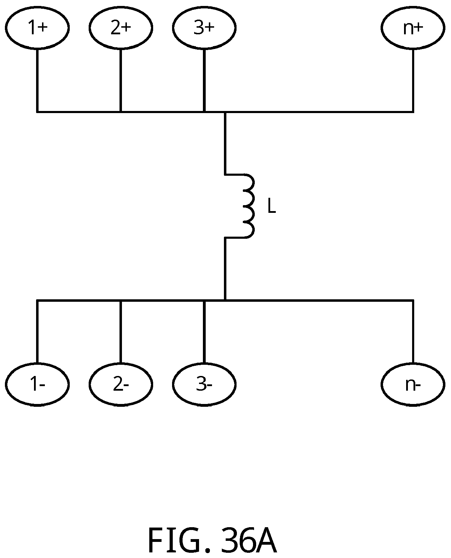

A ninth embodiment of the present invention is as shown in **FIG. 35**, **FIG. 36A** to **FIG. 36C**. The ninth embodiment is an extension of the second embodiment to the eighth embodiment. **FIG. 35** depicts a schematic view of an i^{th} driver circuit of a portable rTMS apparatuses **1** according to an embodiment of the present invention. To simplify the description, elements unrelated to this embodiment have been omitted and not depicted, and other components required during the operation of the driver circuit shall be appreciated by those of ordinary skill in the art based on the aforesaid embodiments and thus will not be further described herein. In this embodiment, two ends of the inductor **L** in the driver circuit (not shown) are distinguished respectively as a positive end and a negative end, **i+** represents the positive connection point of the i^{th} driver circuit, **i-** represents the negative connection point of the i^{th} driver circuit. If the voltage or current of a single driver circuit is insufficient, then a plurality of driving loops may be superimposed to achieve the driving effect.

For example, in the case where the voltage of a single driving loop is sufficient to drive the inductor **L** but the current is insufficient, circuit configuration of **FIG. 36A** may be used, in which n driver circuits are connected in parallel to obtain sufficient current, the positive connection point of each driver circuit is connected to the positive end of the inductor **L**, and the negative connection point of each driver circuit is connected to the negative end of the inductor **L**.

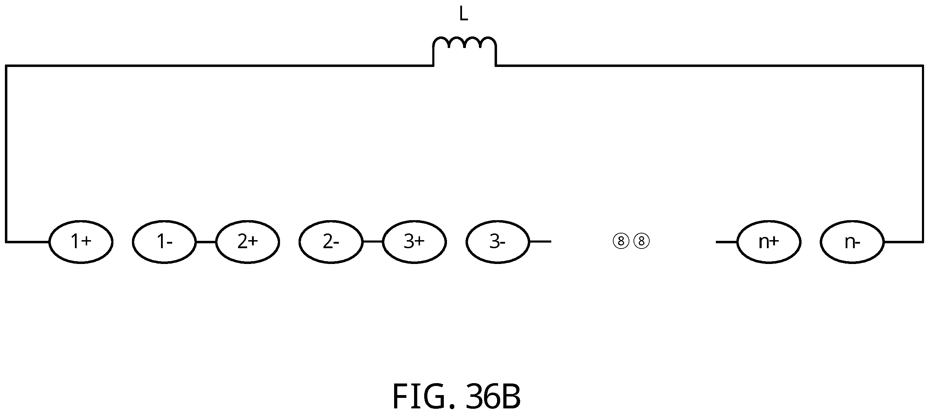

As another example, in the case where the current of a single driving loop is sufficient to drive the inductor **L** but the voltage is insufficient, circuit configuration of **FIG. 36B** may be used, in which a plurality of driver circuits are connected in series to obtain sufficient voltage. In the driver circuit of **FIG. 36B**, only a positive connection point **1+** of the first driver circuit is connected to the positive end of the inductor **L**, and a negative connection point **n-** of the n^{th} driver circuit is connected to the negative end of the inductor **L**. When using the series connection configuration, the passive snubber should still be connected at two sides of the stimulator inductor, and the capacitor **Csn** and the diode **Dsn1** of the energy-recovering snubber still bridge two sides of the inductor, and the diode **Dsn2** and the inductor **Lsn** are connected to the sub-circuit where the capacitor **Csn** is located. If the energy storage capacitor uses the superimposed structure, the sum of the energy storage of the energy storage capacitors of all the sub-circuits should be greater than 2.5 J.

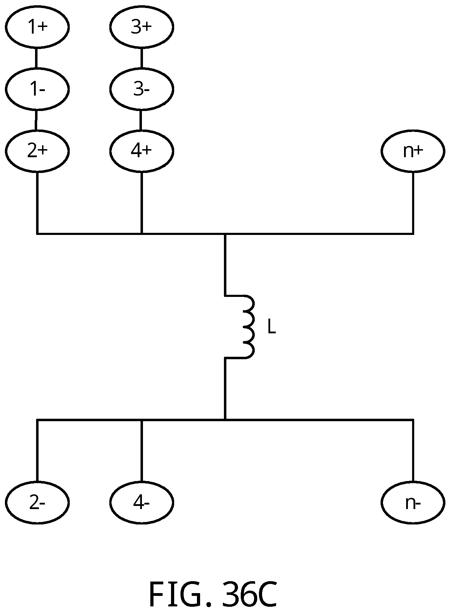

In other embodiments, a plurality of driver circuits may also be partially connected in series and partially connected in parallel to obtain enough current and voltage, as shown in **FIG. 36C**, and the circuit principle thereof is deducted according to the superposition characteristics of a lumped element circuit in the linear system. What described above is the principle of a cascode converter.

It shall be appreciated that, this embodiment takes the driver circuit of the third embodiment for illustration, and the operation of the series connection and parallel connection of the driver circuit of other embodiments shall be appreciated by those of ordinary skill in the art based on the above description, and thus will not be further described herein. In other words, in this embodiment, in addition to the driver circuit of the aforesaid embodiments, a plurality of other driver circuits may be further included, and each of the other driver circuits will be the same as the original driver circuit. Based on different design requirements, these other driver circuits may be connected with the original driver circuit in parallel and then connected to the inductor **L**, or these other driver circuits may be connected with the original driver circuit in series and then connected to the inductor **L**, or these other driver circuits may even have a part thereof connected with the original driver circuit in parallel and another part thereof connected with the original driver circuit in series and then connected to the inductor **L**.

A tenth embodiment of the present invention is as shown in **FIG. 44**. The housing carrying the inductor **L** may be designed to have an appearance similar to an earphone so as to be conveniently fastened on the head. However, in order to fix the position of the stimulator (i.e., the inductor **L**), this new model further provides a fastening device **FD** connected to the housing carrying the inductor **L**, and uses end points of the auricle, the eyes and the fastening device as the positioning points, thereby preventing the position of the stimulator from being shifted during the use thereof. Additionally, in other embodiments, a plurality of stimulators may be disposed by adopting a plurality of inductors **L** that are connected in parallel, and this shall be accomplished by those of ordinary skill in the art based on the aforesaid embodiments, and thus will not be further described herein. With the development of materials in the future, it is more possible to integrate the driver circuit and the stimulator on the head device.

According to the above descriptions, the portable rTMS apparatus of the present invention uses a specially designed stimulator (inductor) and circuit to reduce the power consumption required during the use thereof, improve the heat-dissipating capability and reduce the overall volume of the apparatus, thereby achieving the function of being portable. Accordingly, as compared to the conventional rTMS apparatus, the portable rTMS apparatus of the present invention is not limited by the environment when using, and can be powered by a built-in battery or a mobile power supply.

It shall be appreciated that, these embodiments of the present invention are only for disclosure of implementation contents and technical feature, not for limiting the present invention coverage. Besides, people skilled in this field may proceed with a variety of modifications and replacements based on the disclosures and suggestions without departing from the characteristics, they have substantially been covered in the following claims as appended, and the scope claimed in this application shall be governed by the claims

## Claims

1. A portable repetitive transcranial magnetic stimulation (rTMS) apparatus, comprising:
a driver circuit; and
an inductor, being used as a stimulator, connected to the driver circuit;
wherein the inductor is composed of a core and at least one coil, the core has a groove, the at least one coil has an upper part and a lower part, the upper part of the at least one coil is configured to be distant to the core and pass through an upper side, or a right side, or a left side of the core, and the lower part of the at least one coil is configured to pass through the groove of the core.

2. The portable rTMS apparatus of Claim 1, wherein the upper part of the at least one coil is distant to the core, the groove has a width between 0.7 cm and 11.2 cm, the groove and an average current of the lower part of the at least one coil have a bending angle of less than 60°, and the core has a length between 0.7 cm and 11.2 cm and a thickness between 0.4 cm and 4 cm.

3. The portable rTMS apparatus of Claim 1 or 2, further comprising an upper fastening component and a lower fastening component, wherein the upper part of the at least one coil is further configured to be fastened by the upper fastening component, the lower part of the at least one coil is further configured to be fastened by the lower fastening component, the lower fastening component includes a plurality of ceramic substrates, each of the ceramic substrates has a middle part, a first end and a second end, the first end and the second end are connected by the middle part, and the ceramic substrates are stacked into a layered structure.

4. The portable rTMS apparatus of Claim 3, wherein for any two adjacent ceramic substrates among the ceramic substrates, a size of an upper ceramic substrate is smaller than that of a lower ceramic substrate so that a ladder shape is formed by the first ends and the second ends.

5. The portable rTMS apparatus of Claim 2, wherein the upper part of the at least one coil is more than 7 mm away from the core, the width of the groove is between 1.4 cm and 5.6 cm, the length of the core is between 1.4 cm and 5.6 cm, and the thickness of the core is between 0.7 cm and 2.8 cm.

6. The portable rTMS apparatus of Claim 2 or 5, wherein the groove has a depth between 0.7 cm and 4 cm.

7. The portable rTMS apparatus of Claim 2, wherein the core is composed of a plurality of iron core sheets arranged in a direction perpendicular to a current direction, the core is formed of a high saturation flux density material with a saturation density greater than 1.3 T, and each of the iron core sheets has a thickness less than 1 mm.

8. The portable rTMS apparatus of Claim 2, wherein the core is composed of a plurality of iron core sheets arranged in a direction perpendicular to a current direction, the core is formed of a high saturation flux density material with a saturation density greater than 1.9 T, and each of the iron core sheets has a thickness less than 0.5 mm.

9. The portable rTMS apparatus of Claim 1, wherein the inductor further includes a plurality of extended ceramic substrates that contact with the ceramic substrates respectively along two side portions of the core or are integrated with the ceramic substrates.

10. The portable rTMS apparatus of Claim 1, wherein the inductor further includes an upper blocking component made of a high hardness material, and the core of the inductor is at least partly covered by the upper blocking component.

11. The portable rTMS apparatus of Claim 1, wherein the inductor further includes a lower blocking component made of a high hardness material, and the two side portions of the core of the inductor are connected by the lower blocking component.

12. The portable rTMS apparatus of Claim 11, wherein the lower blocking component further includes a magnetostrictive material.

13. The portable rTMS apparatus of Claim 1, further comprising a housing with high acoustic impedance, wherein the core is disposed inside the housing, the housing and the core are separated by at least a layer of substance having low acoustic impedance therebetween, the layer of substance having low acoustic impedance has a thickness ranging between 0.4 cm and 8 cm, the housing and the core are connected by a spring having a coefficient between 0.001 mm/N and 0.1 mm/N, and the housing has a thickness ranging between 0.5 mm and 1 cm.

14. The portable rTMS apparatus of Claim 2, wherein the driver circuit comprises:
a first energy storage capacitor;
a second energy storage capacitor;
a first switch with freewheeling diode;
a second switch with freewheeling diode;
a first pulse generator coupled to the first switch with freewheeling diode, generating a first pulse signal to control the first switch with freewheeling diode; and
a second pulse generator coupled to the second switch with freewheeling diode, generating a second pulse signal to control the second switch with freewheeling diode;
wherein the inductor, the first energy storage capacitor and the first switch with freewheeling diode are connected in series to form a first driver loop, the inductor, the second energy storage capacitor and the second switch with freewheeling diode are connected in series to form a second driver loop, the first pulse signal and the second pulse signal enable the first driver loop and the second driver loop to conduct alternately, and the first driver loop and the second driver loop have magnetic flux driving directions opposite to each other in respect to the inductor.

15. The portable rTMS apparatus of Claim 2, wherein the driver circuit comprises:
a first energy storage capacitor;
a second energy storage capacitor;
a first switch with freewheeling diode;
a second switch with freewheeling diode;
a first pulse generator coupled to the first switch with freewheeling diode, generating a first pulse signal to control the first switch with freewheeling diode; and
a second pulse generator coupled to the second switch with freewheeling diode, generating a second pulse signal to control the second switch with freewheeling diode;
wherein the inductor, the first energy storage capacitor, the second energy storage capacitor and the first switch with freewheeling diode are connected in series to form a first driver loop, the inductor, the second energy storage capacitor and the second switch with freewheeling diode are connected in series to form a second driver loop, the first pulse signal and the second pulse signal enable the first driver loop and the second driver loop to conduct alternately, and the first driver loop and the second driver loop have magnetic flux driving directions opposite to each other in respect to the inductor.

16. The portable rTMS apparatus of Claim 14 or 15, wherein the driver circuit further comprises at least one passive snubber connected to the first switch with freewheeling diode and the second switch with freewheeling diode or connected to the inductor.

17. The portable rTMS apparatus of Claim 2, wherein the driver circuit comprises:
an energy storage capacitor;
a first switch with freewheeling diode;
a first auxiliary switch with freewheeling diode;
a second switch with freewheeling diode;
a second auxiliary switch with freewheeling diode;
a first pulse generator coupled to the first switch with freewheeling diode and the first auxiliary switch with freewheeling diode, generating a first pulse signal to control the first switch with freewheeling diode and the first auxiliary switch with freewheeling diode; and
a second pulse generator coupled to the second switch with freewheeling diode and the second auxiliary switch with freewheeling diode, generating a second pulse signal to control the second switch with freewheeling diode and the second auxiliary switch with freewheeling diode;
wherein the inductor, the energy storage capacitor, the first switch with freewheeling diode and the first auxiliary switch with freewheeling diode are connected in series to form a first driver loop, the inductor, the energy storage capacitor, the second switch with freewheeling diode and the second auxiliary switch with freewheeling diode are connected in series to form a second driver loop, and the first driver loop and the second driver loop have magnetic flux driving directions opposite to each other in respect to the inductor.

18. The portable rTMS apparatus of Claim 17, wherein the driver circuit further comprises at least one passive snubber connected to the first switch with freewheeling diode, the first auxiliary switch with freewheeling diode, the second switch with freewheeling diode and the second auxiliary switch with freewheeling diode, or connected to the inductor.

19. The portable rTMS apparatus of Claim 2, wherein the driver circuit comprises:
an energy storage capacitor;
a switch;
an auxiliary switch;
a first freewheeling diode;
a second freewheeling diode; and
a pulse generator coupled to the switch and the auxiliary switch, generating a pulse signal to control the switch and the auxiliary switch;
wherein the inductor, the energy storage capacitor, the switch and the auxiliary switch are connected in series to form a driver loop, and the inductor, the first freewheeling diode and the second freewheeling diode are connected in series to form a charging loop.

20. The portable rTMS apparatus of Claim 19, wherein the driver circuit further comprises at least one passive snubber connected to the switch and the auxiliary switch or connected to the inductor.

21. The portable rTMS apparatus of Claim 2, wherein the driver circuit comprises:
a first energy storage capacitor;
a second energy storage capacitor;
a first switch with freewheeling diode;
a second switch with freewheeling diode;
a first pulse generator coupled to the first switch with freewheeling diode, generating a first pulse signal to control the first switch with freewheeling diode; and
a second pulse generator coupled to the second switch with freewheeling diode, generating a second pulse signal to control the second switch with freewheeling diode;
wherein the at least one coil includes a primary coil and a secondary coil, the primary coil, the first energy storage capacitor and the first switch with freewheeling diode are connected in series to form a first driver loop, the secondary coil, the second energy storage capacitor and the second switch with freewheeling diode are connected in series to form a second driver loop, the first pulse signal and the second pulse signal enable the first driver loop and the second driver loop to conduct alternately, and the first driver loop and the second driver loop have magnetic flux driving directions opposite to each other in respect to the inductor.

22. The portable rTMS apparatus of Claim 2, wherein the driver circuit comprises:
an energy storage capacitor;
a first switch with freewheeling diode;
a second switch with freewheeling diode;
a first pulse generator coupled to the first switch with freewheeling diode, generating a first pulse signal to control the first switch with freewheeling diode; and
a second pulse generator coupled to the second switch with freewheeling diode, generating a second pulse signal to control the second switch with freewheeling diode;
wherein the at least one coil includes a primary coil and a secondary coil, the primary coil, the energy storage capacitor and the first switch with freewheeling diode are connected in series to form a first driver loop, the secondary coil, the capacitor and the second switch with freewheeling diode are connected in series to form a second driver loop, the first pulse signal and the second pulse signal enable the first driver loop and the second driver loop to conduct alternately, and the first driver loop and the second driver loop have magnetic flux driving directions opposite to each other in respect to the inductor.

23. The portable rTMS apparatus of Claim 21 or 22, wherein the driver circuit further comprises at least one passive snubber connected to the first switch with freewheeling diode and the second switch with freewheeling diode, or at least one energy-recovering snubber connected to the inductor.

24. The portable rTMS apparatus of Claim 2, wherein the driver circuit comprises:
an energy storage capacitor;
a switch;
a freewheeling diode; and
a pulse generator coupled to the switch, generating a pulse signal to control the switch;
wherein the at least one coil includes a primary coil and a secondary coil, the primary coil, the energy storage capacitor and the switch are connected in series to form a driver loop, and the secondary coil, the energy storage capacitor and the freewheeling diode are connected in series to form a charging loop.

25. The portable rTMS apparatus of Claim 24, wherein the driver circuit further comprises one passive snubber connected to the switch or at least one energy-recovering snubber connected to the inductor.

26. The portable rTMS apparatus of Claim 2, wherein a first number of conductors of the lower part of the coil is smaller than a second number of conductors of the upper part of the coil.

27. The portable rTMS apparatus of Claim 2, wherein the upper part of the coil is in the form of a plate-shaped structure.

28. The portable rTMS apparatus of Claim 2, wherein a cross-sectional area of conductors of the upper part of the coil is larger than a cross-sectional area of conductors of the lower part of the coil.

29. The portable rTMS apparatus of Claim 2, wherein a total conductor spacing of the upper part of the coil is larger than that of the lower part of the coil.
